# EUROPEAN PATENT SPECIFICATION

(11) **EP 2 721 147 B1**
(45) Date of publication and mention of the grant of the patent: **25.04.2018**
(21) Application number: 12799750.0
(22) Date of filing: 15.06.2012
(51) Int. Cl.: C07K 16/28, A61K 39/395, A61P 31/14

(54) **METHODS OF TREATING RETROVIRAL INFECTIONS IN FELINES**
VERFAHREN ZUR BEHANDLUNG RETROVIRALER INFEKTIONEN BEI KLEINKATZEN
MÉTHODES DE TRAITEMENT D'INFECTIONS RÉTROVIRALES CHEZ DES FÉLINS

(30) Priority: 17.06.2011 US 201161498029 P
(43) Date of publication of application: 23.04.2014
(73) Proprietor: CytoDyn, Inc., Vancouver, WA 98660-2970 (US)
(72) Inventor: TRAUGER, Richard, Leucadia, CA 92024 (US)
(74) Representative: Potter Clarkson LLP
(86) International application number: PCT/US2012/042693
(87) International publication number: WO 2012/174392

(56) References cited:
- WO-A1-02/059114
- WO-A2-01/03681
- US-A1- 2002 039 577
- US-A1- 2002 039 577
- C D Rizzuto ET AL: "Contribution of virion ICAM-1 to human immunodeficiency virus infectivity and sensitivity to neutralization", Journal of virology, 1 June 1997 (1997-06-01), pages 4847-4851, XP055139615, UNITED STATES Retrieved from the Internet: URL:http://www.pubmedcentral.nih.gov/artic lerender.fcgi?artid=191712&tool=pmcentrez& rendertype=abstract
- MASAYUKI SHIMOJIMA ET AL: "Phenotypic changes in CD8+ peripheral blood lymphocytes in cats infected with feline immunodeficiency virus", MICROBES AND INFECTION, vol. 5, no. 13, 1 November 2003 (2003-11-01), pages 1171-1176, XP055139613, ISSN: 1286-4579, DOI: 10.1016/j.micinf.2003.08.004
- GEBHARD D H ET AL: "Progressive expansion of an L-selectin-negative CD8 cell with anti-feline immunodeficiency virus (FIV) suppressor function in the circulation of FIV-infected cats.", November 1999 (1999-11), THE JOURNAL OF INFECTIOUS DISEASES NOV 1999, VOL. 180, NR. 5, PAGE(S) 1503 - 1513, XP002741539, ISSN: 0022-1899 * the whole document *
- SHIMOJIMA ET AL.: 'Phenotypic changes in CD8+ peripheral blood lymphocytes in cats infected with feline immunodeficiency virus.' MICROBES AND INFECTION vol. 5, 2003, pages 1171 - 1176, XP055139613
- RIZZUTO ET AL.: 'Contribution of Virion ICAM-1 to Human Immunodeficiency Virus Infectivity and Sensitivity to Neutralization.' JOURNAL OF VIROLOGY vol. 71, no. 6, June 1997, pages 4847 - 4851, XP055139615
- LI ET AL.: 'Efalizumab binding to the LFA-1 alphaL I domain blocks ICAM-1 binding via steric hindrance.' PNAS vol. 106, no. 11, 17 March 2009, pages 4349 - 4354, XP055139616
- DOBSON ET AL.: 'Diagnosis and management of leukaemia in dogs and cats.' PRACTICE vol. 28, 2006, pages 22 - 31, XP008172913
- PETRUZZELLI ET AL.: 'Activation of Lymphocyte Function-Associated Molecule (CDlla/CD18) and Mac -1 (CD11 b/CD18) Mimicked by an Antibody Directed Against CD18.' JOURNAL OF IMMUNOLOGY vol. 155, no. 2, 1995, pages 854 - 866, XP002209615
- RICE ET AL.: 'INDUCIBLE CELL ADHESION MOLECULE 110 (INCAM-110) IS AN ENDOTHELIAL RECEPTOR FOR LYMPHOCYTES.' J.EXP.MED. vol. 171, April 1990, pages 1369 - 1374, XP055139617

## Description

### TECHNICAL FIELD

This invention relates generally to methods of treating retroviral infections in felines.

### BACKGROUND

Felines are susceptible to infection by retroviruses (e.g., feline immunodeficiency virus (FIV) and feline leukemia virus (FELV)). FIV is the causative agent of feline immunodeficiency disease syndrome. FELV is caused by a feline retrovirus that is similar to human leukemia virus in humans. Feline leukemia virus induces the uncontrolled growth of blood cells.

Feline retroviruses affect a significant portion of the feline population. For example, approximately 2.5% to 4.4% of cats worldwide were estimated to be infected with FIV in 2005 (Richards, Biologicals 33:219, 2005). Approximately 0.5% of domestic felines are persistently infected with FELV. Successful treatment of FIV and FELV infection in felines (e.g., domestic and feral felines) is desired.

Rizzuto et al (J of Virology 71(6):4847-4851) describes the contribution of virion ICAM-1 to human immunodeficiency virus infectivity and sensitivity to neutralisation.

Shimojima et al (Microbes and Infection 5:1171-1176) describes phenotypic changes in CD8⁺ peripheral blood lymphocytes in cats infected with feline immunodeficiency virus.

### SUMMARY

The present invention is based, at least in part, on the discovery that an antibody that specifically binds to the cellular antigen LFA-1 (CD11a/CD18 heterodimer) reduces retroviral infection of feline cells in vitro. The present invention is as defined in the claims and is directed at an antibody or antigen-binding fragment thereof that specifically binds to CD11a, for use in treating a retrovirus in a feline. To the extent that other subject matter is provided or described herein, it is included as disclosures merely for reference purposes. Provided herein are methods of treating retroviral infections in felines by administering one or more agents that specifically bind to CD11a, CD18, an epitope formed by both CD11a and CD18, or ICAM-1 present on the surface of a feline cell (e.g., a non-infected feline cell) or present on the surface of a retroviral virion or syncytium. In some embodiments, the one or more agents decrease the binding of LFA-1 to ICAM-1. Also provided are
methods of reducing or preventing retroviral virion entry into a feline cell (e.g., a non-infected feline cell) or reducing or preventing retroviral virion budding from a feline cell by administering at least one agent that specifically binds to CD11a, CD18, an epitope formed by both CD11a and CD18, or ICAM-1, and/or decreases the binding of LFA-1 to ICAM-1. Also provided are methods of reducing or preventing syncytium transmission in a feline by administering at least one agent that specifically binds to CD11a, CD18, an epitope formed by both CD11a and CD18, or ICAM-1, and/or prevents or decreases LFA-1 binding to ICAM-1.

In some embodiments of all of the methods described herein, the at least one agent decreases the interaction (e.g., decreases the Kₒₙ rate, increases the K_{off} rate, increases the K_{D}, and/or decreases the K_{A}) of LFA-1 with ICAM-1. The LFA-1 can be present on the surface of a feline cell (e.g., a non-infected feline cell) with ICAM-1 present on the surface of a retroviral virion or syncytium. In some embodiments, the LFA-1 can be present on the surface of a retroviral virion or syncytium with ICAM-1 present on the surface of a feline cell (e.g., a non-infected feline cell). In some embodiments, the at least one agent decreases the ability of LFA-1 or ICAM-1 to contribute to vision entry into a feline cell (e.g., an non-infected feline cell) or decreases the ability of LFA-1 or ICAM-1 to contribute to virion budding. In some embodiments, the at least one agent decreases the ability of LFA-1 or ICAM-1 to contribute to syncytium transmission in a feline. In any of the embodiments described herein, LFA-1 may be present on the surface of a feline cell (e.g., a non-infected feline cell) or on the surface of a retroviral virion or syncytium. In any of the embodiments described herein, ICAM-1 may be present on the surface of a feline cell (e.g., a non-infected feline cell) or on the surface of a retroviral vision or syncytium.

Compositions containing at least one agent that specifically binds to CD 11a, CD18, an epitope formed by both CD11a and CD18, or ICAM-1, and/or decreases the interaction of LFA-1 with ICAM-1 are also provided, as well as methods of identifying such agents.

Provided are methods for treating a retrovirus infection in a feline. These methods include administering to a feline at least one (e.g., one, two, three, or four) small molecule that decreases LFA-1 binding to ICAM-1, where the at least one small molecule is administered in an amount sufficient to treat a retrovirus infection. Also provided are methods for treating a retrovirus infection in a feline that include administering to a feline at least one (e.g., one, two, three, or four) antibody or antigen-binding fragment thereof that specifically binds to CD11a and/or CD18 (binds specifically to CD11a, CD18, or an epitope that is formed by both CD11a and CD18), wherein the at least one antibody or antigen-binding fragment thereof is administered in an amount sufficient to treat a retrovirus infection. Also provided are methods for treating a retrovirus infection in a feline that include administering to a feline at least one (e.g., one, two, three, or four) antibody or antigen-binding fragment thereof that specifically binds to ICAM-1, wherein that at least one antibody or antigen-binding fragment thereof is administered in an amount sufficient to treat a retrovirus infection.

Also provided are methods for reducing retroviral virion entry into a feline cell (e.g., a non-infected feline cell) or retroviral virion budding from a feline cell in a feline. These methods include administering to a feline at least one (e.g., one, two, three, or four) small molecule that decreases LFA-1 binding to ICAM-1, wherein the at least one (e.g., one, two, three, or four) small molecule is administered in an amount sufficient to reduce retroviral virion entry into a feline cell or retroviral vision budding from a feline cell. Also provided are methods for reducing retroviral virion entry into a feline cell or retroviral virion budding from a feline cell in a feline that include administering at least one (e.g., one, two, three, or four) antibody or antigen-binding fragment thereof that specifically binds to CD11a and/or CD 18 (binds specifically to CD11a, CD18, or an epitope formed by both CD11a and CD18), wherein the at least one antibody or antigen-binding fragment thereof is administered in an amount sufficient to reduce retroviral virion entry into a feline cell or retroviral vision budding from a feline cell. Also provided are methods for reducing retroviral virion entry into a feline cell or retroviral virion budding from a feline cell in a feline that include administering at least one (e.g., one, two, three, or four) antibody or antigen-binding fragment thereof that specifically binds to ICAM-1, wherein the at least one antibody or antigen-binding fragment thereof is administered in an amount sufficient to reduce retroviral virion entry into a feline cell or retroviral virion budding from a feline cell.

Also provided are methods of reducing syncytium transmission in a feline. These methods include administering to a feline at least one (e.g., one, two, three, or four) small molecule that decreases LFA-1 binding to ICAM-1, wherein the at least one small molecule is administered in an amount sufficient to reduce syncytium transmission in a feline. Also provided are methods of reducing syncytium transmission in a feline that include administering to a feline at least one (e.g., one, two, three, or four) antibody or antigen-binding fragment thereof that specifically binds to CD11a and/or CD18 (binds specifically to CD11a, CD18, or an epitope formed by both CD11a and CD18), wherein the at least one antibody or antigen-binding fragment thereof is administered in an amount sufficient to reduce syncytium transmission in a feline. Also provided are methods of reducing syncytium transmission in a feline that include administering to a feline at least one (e.g., one, two, three, or four) antibody or antigen-binding fragment thereof that specifically binds to ICAM-1, where the at least one antibody or antigen-binding fragment thereof is administered in an amount sufficient to reduce syncytium transmission in a feline.

In some embodiments, the at least one antibody or antigen-binding fragment thereof binds specifically to CD11a and/or CD18 (binds specifically to CD11a, CD18, or an epitope formed by both CD11a and CD18), or ICAM-1. In some embodiments, the at least one antibody or antigen-binding fragment thereof decreases LFA-1 binding to ICAM-1. In any of the methods described herein, the at least one antibody is a monoclonal antibody.

In some embodiments, the at least one antibody or antigen-binding fragment thereof that specifically binds to CD11a is an antibody or an antigen-binding fragment thereof that contains at least one complementary determining region (CDR) from the light chain or the heavy chain of TS1/22 antibody (produced from ATCC deposit number HB202). In some embodiments, the at least one antibody or antigen-binding fragment thereof is an antibody or an antigen-binding fragment thereof that contains the three CDRs from the light chain of TS1/22 antibody or the three CDRs from the heavy chain of TS1/22 antibody. In some embodiments, the at least one antibody or antigen-binding fragment thereof is an antibody or an antigen-binding fragment thereof that contains the three CDRs from the light chain of TS1/22 antibody and the three CDRs from the heavy chain of TS1/22 antibody.

In some embodiments, the at least one antibody or antigen-binding fragment thereof that specifically binds to CD11a is an antibody or an antigen-binding fragment thereof that contains at least one complementary determining region (CDR) from the light chain or the heavy chain of clone 25.3 antibody (Becker Coulter (PN IM0157 and PN IM1433U). In some embodiments, the at least one antibody or antigen-binding fragment thereof is an antibody or an antigen-binding fragment thereof that contains the three CDRs from the light chain of clone 25.3 antibody or the three CDRs from the heavy chain of clone 25.3 antibody. In some embodiments, the at least one antibody or antigen-binding fragment thereof is an antibody or an antigen-binding fragment thereof that contains the three CDRs from the light chain of clone 25.3 antibody and the three CDRs from the heavy chain of clone 25.3 antibody.

In some embodiments, the at least one antibody or antigen-binding fragment thereof that specifically binds to CD11a is an antibody or an antigen-binding fragment thereof that contains at least one complementary determining region (CDR) from the light chain or the heavy chain of clone 27 antibody (Fisher Scientific No. 610826). In some embodiments, the at least one antibody or antigen-binding fragment thereof is an antibody or an antigen-binding fragment thereof that contains the three CDRs from the light chain of clone 27 antibody or the three CDRs from the heavy chain of clone 27 antibody. In some embodiments, the at least one antibody or antigen-binding fragment thereof is an antibody or an antigen-binding fragment thereof that contains the three CDRs from the light chain of clone 27 antibody and the three CDRs from the heavy chain of clone 27 antibody.

In some embodiments, the at least one antibody or antigen-binding fragment thereof is TS1/22 antibody or an antigen-binding fragment of TS1/22 antibody. In some embodiments, the at least one antibody or antigen-binding fragment is clone 25.3 antibody or an antigen-binding fragment of a clone 25.3 antibody. In some embodiments, the at least one antibody or antigen-binding fragment is a clone 27 antibody or an antigen-binding fragment of a clone 27 antibody.

In some embodiments, the at least one antibody is a feline antibody or an antigen-binding fragment thereof, or a felinized antibody or an antigen-binding fragment thereof. In some embodiments of all of the methods described herein, the antibody is a single-chain antibody (e.g., a single-domain antibody). In some embodiments, the at least one antigen-binding fragment thereof is selected from the group consisting of: a Fab fragment, a F(ab')₂ fragment, and a scFv fragment. In some embodiments, the at least one small molecule is lovastatin, simvastatin, a lovastatin derivative (e.g., a 6-exomethylene-modified, an 8-acyl-modified, or an alkylated lovastatin), a simvastatin, derivative, LFA703, LFA451, LFA878, or XVA143, or a salt, solvate, or hydrate thereof.

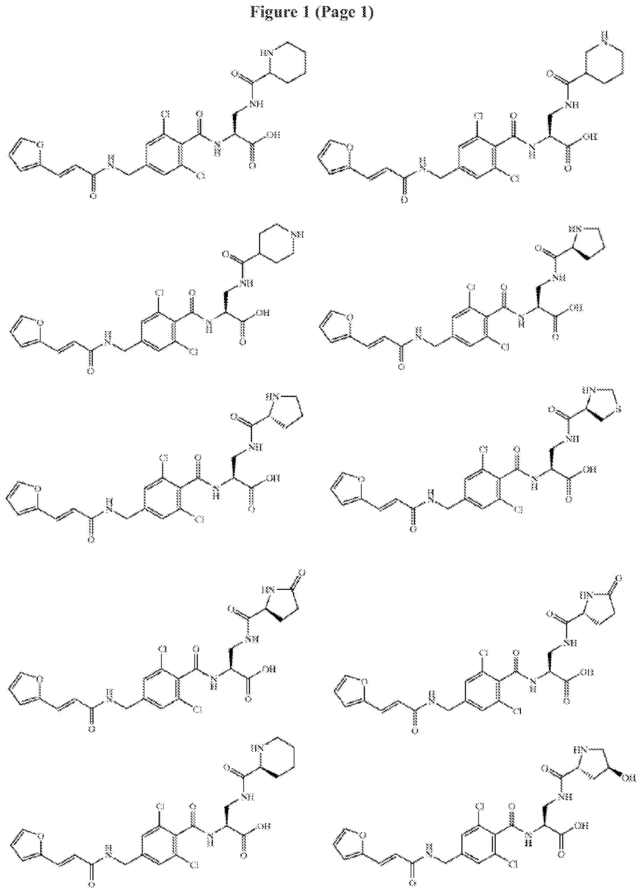

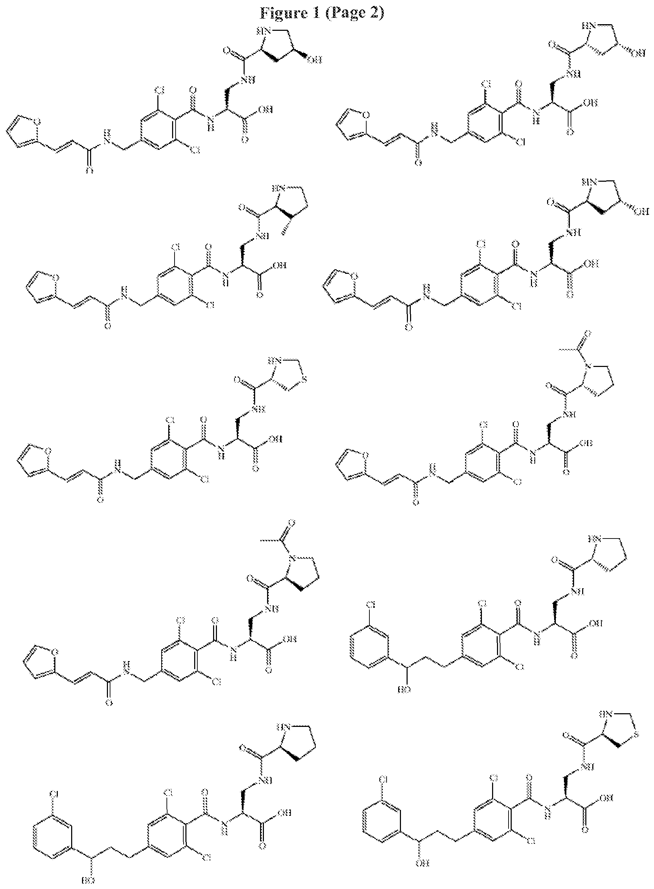

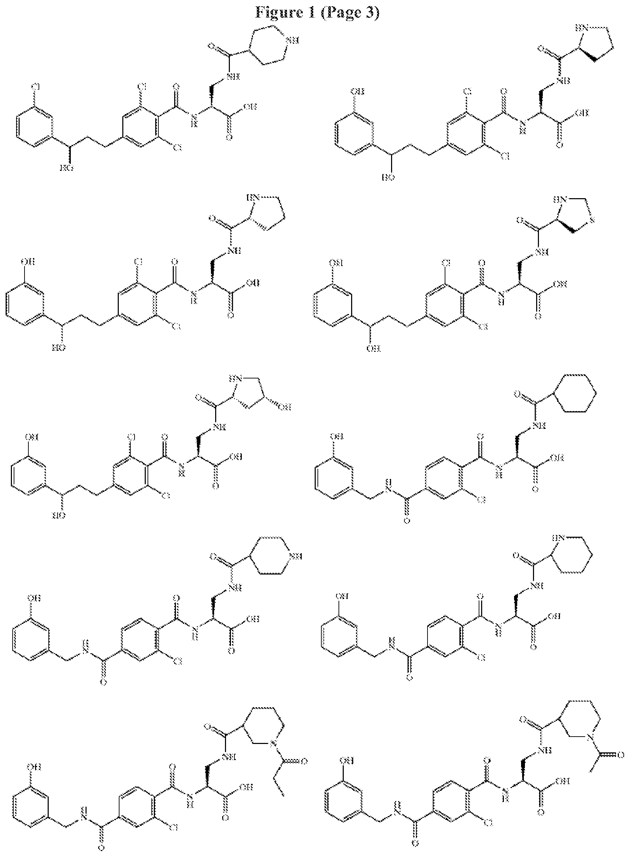

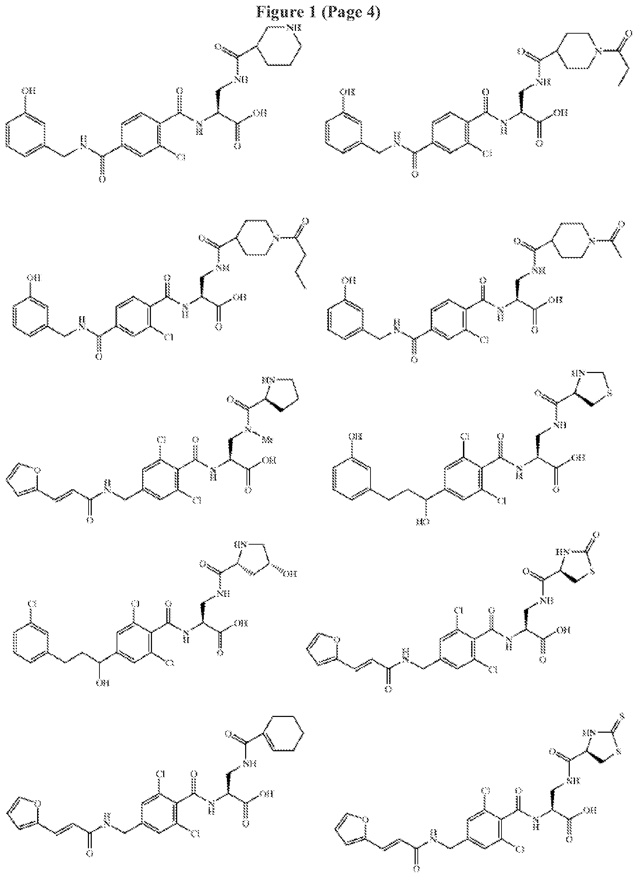

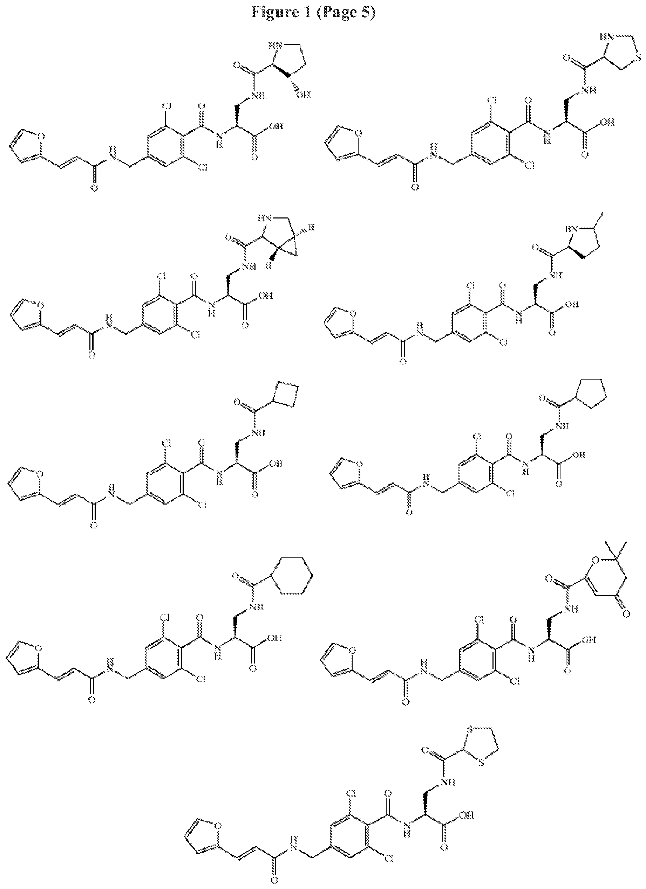

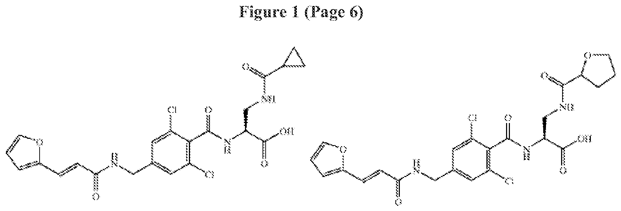

In some embodiments, the at least one small molecule is: 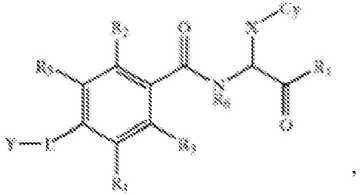 where
Cy is a non-aromatic carbocycle or heterocycle, optionally substituted with hydroxyl, mercapto, thioalkyl, halogen, oxo, thio, amino, aminoalkyl, amidine, guanidine, nitro, alkyl, alkoxy, or acyl; X is a divalent hydrocarbon chain, optionally substituted with hydroxy, mercapto, halogen, amino, aminoalkyl, nitro, oxo, or thio, and optionally interrupted with N, O, S, SO, or SO₂; Y is a carbocycle or heterocycle, optionally substituted with hydroxyl, mercapto, halogen, oxo, trio, thioalkyl, amino, aminoalkyl, carbocycle or heterocycle ring, hydrocarbon, a halo-substituted hydrocarbon, amino, amidine, guanidine, cyano, nitro, alkoxy, or acyl; L is a divalent hydrocarbon chain, optionally substituted with hydroxyl, halogen, oxo, or thio; R₁ is H, OH, amino, O-carbocycle, or alkoxy, optionally substituted with amino, a carbocycle or heterocycle; R₂₋₅ are independently H, hydroxyl, mercapto, halogen, cyano, amino, amidine, guanidine, nitro, or alkoxy; or R₃ or R₄ together form a fused carbocycle or heterocycle, optionally substituted with hydroxyl, halogen, oxo, thio, amino, amidine, guanidine, or alkoxy; and R₆ is H or a hydrocarbon chain, optionally substituted with a carbocycle or a heterocycle. In some embodiments, at least one small molecule can be any of the molecules shown in Figure 1. Any of the small molecules described herein can be in the form of a salt, solvate, or hydrate.

In some embodiments, the at least one small molecule and/or the at least one antibody or antigen-binding fragment thereof is administered intravenously, intraarterially, ocularly, orally, subcutaneously, intraperitoneally, or intramuscularly. In some embodiments, the at least one small molecule is administered orally. In some embodiments, the at least one antibody or antigen-binding fragment thereof is administered intravenously.

In some embodiments where the feline is administered at least one antibody or antigen-binding fragment thereof (e.g., any of the antibodies or antigen-binding fragment described herein), the feline is further administered at least one (e.g., one, two, three, or four) small molecule (e.g., any of the small molecules described herein) that prevents LFA-1 from binding to ICAM-1. In some embodiments, the at least one small molecule and the at least one antibody or antigen-binding fragment thereof are administered in the same composition. In some embodiments, the at least one small molecule and the at least one antibody or antigen-binding fragment thereof are administered by different routes of administration.

In some embodiments of any of the methods described herein, the administering results in a decrease (e.g., a significant or observable decrease) in the severity, frequency, or duration of at least one symptom of retroviral infection in the feline. In some embodiments of any of the methods described herein, the administering results in a decrease (e.g., a statistically significant decrease) in retroviral titer in the feline. In some embodiments of any of the methods described herein, the administering results in an increase (e.g., a statistically significant increase) in the ratio of CD4⁺ T-cells to CD8⁺ T-cells in the feline. In some embodiments of any of the methods described herein, the at least one small molecule and/or the at least one antibody and/or antigen-binding fragment thereof is administered to the feline at least once a week (e.g., at least once a day). In some embodiments of any of the methods described herein, the administering does not result in detrimental immunosuppression in the feline.

In any of the methods described herein, the retroviral infection is feline immunodeficiency virus (FIV) or feline leukemia virus (FELV). In some embodiments of any of the methods described herein, the retroviral virion is feline immunodeficiency virus (FIV) or feline leukemia virus (FELV).

Also provided herein are methods of using at least one antibody or antigen-binding fragment thereof that specifically binds to CD11a and/or CD18 (e.g.., any of the exemplary antibodies or antibody fragments described herein) in the manufacture of a medicament for treating retrovirus infection in a feline, reducing retrovirus virion entry into a feline cell or retrovirus virion budding from a feline cell in a feline, and/or reducing syncytium transmission in a feline.

Also provided herein are antibodies or antigen-binding fragments thereof that specifically bind to CD11a and/or CD18 for use in treating retrovirus infection in a feline, reducing retrovirus virion entry into a feline cell or retrovirus vision budding from a feline cell in a feline, and/or reducing syncytium transmission in a feline.

Also provided herein are methods of using at least one small molecule that decreases LFA-1 binding to ICAM-1 (e.g., any of the small molecules that decrease LFA-1 binding to ICAM-1 described herein) in the manufacture of a medicament for treating retrovirus infection in a feline, reducing retrovirus virion entry into a feline cell or retrovirus virion budding from a feline cell in a feline, and/or reducing syncytium transmission in a feline.

Also provided herein are small molecules that decrease LFA-1 binding to ICAM-1 for use in treating retrovirus infection in a feline, reducing retrovirus virion entry into a feline cell or retrovirus virion budding from a feline cell in a feline, and/or reducing syncytium transmission in a feline.

Also provided herein are methods of using at least one antibody or antigen-binding fragment thereof that specifically binds to ICAM-1 (e.g., any of the exemplary antibodies or antigen-binding fragments thereof that specifically bind to ICAM-1 described herein) in the manufacture of a medicament for treating retrovirus infection in a feline, reducing retrovirus virion entry into a feline cell or retrovirus virion budding from a feline cell in a feline, and/or reducing syncytium transmission in a feline.

Also provided herein are antibodies or antigen-binding fragments thereof that specifically bind to ICAM-1 for use in treating retrovirus infection in a feline, reducing retrovirus virion entry into a feline cell or retrovirus virion budding from a feline cell in a feline, and/or reducing syncytium transmission in a feline.

By the term "retrovirus infection" or "retrovirus infection in a feline" is meant a disease in a feline where the causative agent is a retrovirus Non-limiting examples of feline retroviruses are described herein.

By the term "lymphocyte function associated antigen-1" or "LFA -1" is meant a heterodimer of CD11a and CD18. LFA-1 plays a role in lymphocyte adhesion and activation leading to a normal immune response. Intercellular adhesion molecules (ICAMs) -1, -2, and -3 are ligands for LFA-1 expressed in the endothelium, leukocytes, and other cell types. As used herein, CD11a, CD18, and LFA-1 refer to feline CD11a, feline CD18, and feline LFA-1.

By the term "intracellular adhesion molecule-1" or "ICAM-1" is meant a cell surface adhesion receptor that is a member of the immunoglobulin protein super-family. ICAM-1 is expressed on a variety of hematopoietic and non-hematopoeitic cells and is up-regulated at sites of inflammation by a variety of inflammatory mediators. ICAM-1 binds to several different cellular receptors, including LFA-1. As used herein, ICAM-1 refers to feline ICAM-1.

By the term "small molecule" is meant any small organic (e.g., peptides, nucleotides, sugars, and/or lipids), small inorganic molecules (e.g., metal complexes), or small organic/inorganic complexes (e.g., metal/protein complexes). In some embodiments, the small molecule can bind directly to CD11a and/or CD18 (bind to CD11a, CD18, or both CD11a and CD18), or ICAM-1. In some embodiments, the small molecule can bind indirectly to CD11a and/or CD18 or ICAM-1. In some embodiments, the small molecule can prevent or decrease LFA-1 binding to ICAM-1.

By the term "antibody" is meant any immunoglobulin or antibody (e.g., human, feline, mouse, cartilagenous fish, or camelid antibodies), and any derivative or conjugate thereof, that specifically binds to an antigen. A wide variety of antibodies are known by those skilled in the art. Non-limiting examples of antibodies include: monoclonal antibodies (e.g., including full-length antibodies), polyclonal antibodies, multi-specific antibodies (e.g., bi-specific antibodies), single-chain antibodies (e.g., single-domain antibodies, camelid antibodies, and cartilagenous fish antibodies), chimeric antibodies, feline antibodies, and felinized antibodies. The term antibody also includes antibody derivatives and conjugates (e.g., an antibody conjugated to a stabilizing protein, a detectable moiety, or a therapeutic agent).

By the term "antigen-binding fragment" is meant any portion of a full-length antibody that contains at least one variable domain (e.g., a variable domain of a mammalian e.g., feline, human, or mouse) heavy or light chain immunoglobulin), a camelid variable antigen-binding domain (VHH), and a cartilagenous fish immunoglobulin new antigen receptor (Ig-NAR) domain) that is capable of specifically binding to an antigen. Non-limiting examples of antibody fragments include Fab, Fab', F(ab')₂, and Fv fragments, diabodies, linear antibodies, and multi-specific antibodies formed from antibody fragments. Additional antibody fragments containing at least one camelid VHH domain or at least one cartilagenous fish Ig-NAR domain include mini-bodies, micro-antibodies, subnano-antibodies, and nano-antibodies, and any of the other forms of antibodies described in WO 2010/033913 A1.

By the phrase "reduces LFA-1 binding to ICAM-1" is meant a statistically significant decrease (e.g., a detectable decrease, e.g., at least 10%, 15%, 20%, 25%, 30%, 35%, 40%, 45%, 50%, 55%, 60%, 65%, 70%, 75%, 80%, 85%, 90%, or 95% decrease) in the binding of LFA-1 to ICAM-1. The reduction in binding of LFA-1 binding to ICAM-1 can be compared to the amount of binding observed in the absence of treatment (e.g., in the absence of any agent as described herein, e.g., an antibody, antigen-binding fragment of an antibody, or small molecule). The binding of LFA-1 to ICAM-1 may be determined, for example, using in vitro cell culture assays or in vitro protein assays using purified proteins (e.g., BioCore technology) or cell lysates. Additional methods for determining binding of LFA-1 to ICAM-1 are known in the art. As used throughout the specification and claims, the phrase "LFA-1 binding to ICAM-1" and "ICAM-1 binding to LFA-1" is used interchangeably.

By the term "retrovirus virion" is meant a single infectious particle of a retrovirus (e.g., a feline retrovirus). A retroviral virion can contain an envelope derived from the plasma membrane of the host feline cell. Retroviral virion contain structural proteins that form a capsid that encloses a nucleic acid encoding the retroviral genome. As is known in the art, the structure of a retroviral virion and its components can vary between different feline retroviruses. In some embodiments, the retroviral virion can contain or present on its surface at least one of CD11a, CD18, or ICAM-1. The CD11a and CD18 present on the surface of a retroviral virion can form LFA-1 (a heterodimer of CD11a and CD18). The CD 11a, CD18, or ICAM-1 contained or presented on the surface of the retroviral virion can contribute or play an active role in the entry (e.g., fusion) of the retroviral virion into a feline cell (e.g., a non-infected feline cell) or the budding or release of a retroviral virion into the extracellular space from an infected feline cell.

By the term "syncytium" is meant a large cell-like structure that contains cytoplasm, at least two nuclei, and an infectious retrovirus. A syncytium can express on its surface (plasma membrane) at least one of CD11a, CD18, or ICAM-1. CD11a and CD 18 on the surface of a syncytium may form LFA-1 (a heterodimer of CD11a and CD18). The CD11a, CD18, or ICAM-1 present on the surface of the syncytium can contribute or play an active role in the fusion of the syncytium with a feline cell (e.g., a non-infected feline cell). The fusion (transient or permanent fusion) of a syncytium with a non-infected feline cell can promote the transmission of a retrovirus.

By the term "syncytium transmission" is meant the transfer of an infectious retrovirus from a syncytium to a non-infected feline cell that is mediated by fusion (e.g., transient or permanent fusion) of a syncytium with a non-infected feline cell. In some non-limiting embodiments, syncytium transmission can result in the transfer of a retroviral virion or capsid to a non-infected feline cell. A syncytium can fuse (e.g., transiently or permanently) with the plasma membrane of a non-infected target cell. Syncytial fusion with a non-infected target cell can be measured using any of the methods known in the art, including microscopic studies. A decrease in syncytium transmission in a feline can be detected by observing a reduction or a delay in the onset of retrovirus-induced dementia in a feline.

By the term "budding" is meant the release of retroviral virion from an infected host cell that includes the step of enveloping a retroviral capsid in the plasma a membrane of the infected host cell.

By the term "entry" is meant the natural introduction of at least one retroviral vision into a non-infected feline cell. Entry of a retroviral virion into a non-infected feline cell can include the step of fusion of a retroviral envelope (enclosing the retroviral capsid) with the plasma membrane of a non-infected feline cell. Retroviral virion entry can be measured using any of the methods known in the art, including microscopic studies.

By the term "feline antibody" is meant an antibody that is encoded by a nucleic acid (e.g., rearranged feline immunoglobulin heavy or light chain locus) present in the genome of a feline. In some embodiments, a feline antibody is produced by a feline or in a feline cell culture (e.g., feline hybridoma cells). In some embodiments, a feline antibody is produced in a non-feline cell (e.g., a mouse or human cell line). In some embodiments, a feline antibody is produced in a bacterial or yeast cell.

By the term "felinized antibody" is meant a feline antibody which contains minimal sequence derived from non-feline (e.g., mouse or human) immunoglobulin. In non-limiting examples, felinized antibodies are feline antibodies (recipient antibody) in which hypervariable region residues of the recipient antibody are replaced by hypervariable region residues from a non-feline species antibody (donor antibody), e.g., mouse, rat, rabbit, or human antibody having the desired specificity, affinity, and capacity. In some embodiments, the Fv framework residues of the feline immunoglobulin are replaced by corresponding non-feline residues. In some embodiments, felinized antibodies may contain residues which are not found in the recipient antibody or in the donor antibody. These modifications can be made to further refine antibody performance.

In some embodiments, the felinized antibody will contain substantially all of at least one, and typically two, variable domains, in which all or substantially all of the hypervariable loops (complementary determining regions) correspond to those of a non-feline immunoglobulin and all or substantially all of the framework regions are those of a feline immunoglobulin sequence. The felinized antibody can also contain at least a portion of an immunoglobulin constant region (Fc), typically, that of a feline immunoglobulin. Felinized antibodies can be produced by molecular biology methods known in the art. Non-limiting examples of methods for generating felinized antibodies are described herein.

By the term "single-chain antibody" is meant a single polypeptide that contains at least one variable binding domain (e.g., a variable domain of a mammalian heavy or light chain immunoglobulin, a camelid variable antigen-binding domain (VHH), or a cartilagenous fish (e.g., shark) immunoglobulin new antigen receptor (Ig-NAR) domain) that is capable of specifically binding to an antigen. Non-limiting examples of single-chain antibodies are described herein, and include single-domain antibodies (described herein).

By the term "single-domain antibody" is meant a polypeptide that contains one camelid variable antigen-binding domain (VHH) or at least one cartilagenous fish (e.g., shark) immunoglobulin new antigen receptor (Ig-NAR) domain that is capable of specifically binding to an antigen. Non-limiting examples of single-domain antibodies are described herein and are known in the art (see, for example, the antibodies described in U.S. Patent Publication No. 2010/0092470).

An antibody or antigen-binding fragment thereof "specifically binds" to a particular antigen, e.g., CD11a and/or CD18 (an epitope on CD11a, CD18, or an epitope that is formed by both CD11a and CD18), or ICAM-1, when it binds to that antigen, but recognizes and binds to a lesser extent (e.g., does not recognize and bind) to other molecules in a sample. In some embodiments, an antibody or an antigen-binding fragment thereof selectively binds to an epitope with an affinity (K_{D}) equal to or less than 1x 10⁻⁷ M (e.g., less than 1 x 10⁻⁸ M or less than 1 x 10⁻⁹ M) in phosphate buffered saline. The ability of an agent or molecule to specifically bind a second agent or molecule may be determined using any of the methods known in the art or those methods described herein.

By the phrase "decrease in the severity, frequency, or duration of at least one symptom" is meant a detectable or observable decrease in the intensity or clinical scoring of at least one symptom of a retroviral infection in a feline, a detectable or observable decrease in the recurrence of at least one symptom of a retroviral infection in a feline, or a detectable or observable decrease in the duration of at least one symptom of a retroviral infection in a feline. Symptoms of a retroviral infection in a feline may be detected, observed, or scored by a veterinary professional or any other individual (e.g., an owner of a domestic feline). A decrease in the severity, frequency, or duration of at least one symptom of a retroviral infection in a feline receiving a treatment (e.g., administered at least one of the agents described herein) may be compared to the severity, frequency, or duration of at least one symptom of a retroviral infection in a control feline (e.g., a feline having the same retroviral infection not receiving treatment or the same feline prior to treatment).

By the term "symptom of retroviral infection" is meant any observable or detectable physiological event that is significantly correlated with a retroviral infection in a feline. A symptom of retroviral infection may be observed by a veterinary professional or a symptom of retroviral infection may be detected by laboratory testing. Non-limiting examples of symptoms of retroviral infection in a feline include: decreased levels of CD4⁺ T-cells (e.g., relative to a control feline or the same feline prior to infection), decreased ratio of CD4⁺ T-cells to CD8⁺ T-cells (e.g., relative to a control feline or the same feline prior to infection), decreased total white cell count (e.g., relative to a control feline or the same feline prior to infection), depression, lack of apetite, discharge from eyes and nose, fever, still and painful joints and muscles, difficulty breathing, sores in the mouth or on the lips, tongue, feet, or nose, weight loss, diarrhea, enlarged lymph nodes, skin infection, bladder infection, upper respiratory infection, seizures, vomiting, bloody diarrhea, weakness, conjunctivitis, stomatitis, odontoclasia, periodontitis, gingivitis, dementia, rhinitis, pneumonitis, enteritis, and dermatitis. Another symptom of a retroviral infection is uncontrolled proliferation of blood cells (feline leukemia). Additional symptoms of retroviral infection in a feline are described herein and are known in the art.

By the term "lovastatin derivative" is meant a modified lovastatin (a modified form of (1S,3R,7S,8S,8aR)-8-{2-[(2R,4R)-4-hydroxy-6-oxooxan-2-yl]ethyl}-3,7-dimethyl-1,2,3,7,8,8a-hexahydronaphthalen-1-yl (2S)-2-methylbutanoate) that has one or more of the following activities: ability to bind (e.g., K_{D} equal to or less than 1 x 10⁻⁶ M) to CD11a, CD18, ICAM-1, or both CD11a and CD18, or the ability to decrease binding of LFA-1 to ICAM-1. Non-limiting examples of lovastatin derivatives are described herein. Additional lovastatin derivatives are known in the art.

By the term "simvastatin derivative" is meant a modified simvastatin, (a modified form of (1S,3R,7S,8S,8aR)-8-(2-((2R,4R)-4-hydroxy-6-oxotetrahydro-2H-pyran-2-yl)ethyl)-3,7-dimethyl-1,2,3,7,8,8a-hexahydronaphthalen-1-yl 2,2-dimethylbutanoate) that has one or more of the following activities: ability to bind to CD11a, CD18, ICAM-1, or both CD11a and CD18 (e.g., K_{D} equal to or less than 1 x 10⁻⁶ M), or the ability to decrease binding of LFA-1 to ICAM-1. Non-limiting examples of simvastatin derivatives are described herein. Additional examples of simvastatin, derivatives are known in the art.

By the term "complementary determining region" or "CDR" is meant a region within an immunoglobulin (heavy or light chain immunoglobulin) that forms part of an antigen-binding site in an antibody or antigen-binding fragment thereof. As is known in the art, a heavy chain immunoglobulin contains three CDRs: CDR1, CDR2, and CDR3, respectively, and a light chain immunogloblun contains three CDRs: CDR1, CDR2, and CDR3. In any antibody or antigen-binding fragment thereof, the three CDRs from the heavy chain immunoglobulin and the three CDRs from the light chain immunoglobulin together form an antigen-binding site in the antibody or antigen-binding fragment thereof. The Kabat Database is one system used in the art to number CDR sequences present in a light chain immunoglobulin or a heavy chain immunoglobulin.

By the term "feline" is meant any mammal belonging to the family Felidae. Non-limiting examples of felines include domestic cats, feral cats, jaguars, lions, and tigers.

By the term "non-aromatic" refers to carbocycle or heterocycle rings that do not have the properties of aromaticity. Aromaticity requires a ring to be planar, have π-orbitals that are perpendicular to the plane of the ring at each ring atom and satisfy the Huckel rule, where the number of π electrons in the ring is (4n + 2) and n is an integer (i.e., the number of π electrons is 2, 6, 10, or 14). Non-aromatic rings do not satisfy one or all of these criteria for aromaticity.

By the term "alkoxy" is meant O-alkyl, O-alkenyl, or an O-alkynyl group. Non-limiting examples of alkoxy groups include methoxy, ethoxy, propoxy, butoxy, i-butoxy, s-butoxy, t-butoxy, pentyloxy, and hexyloxy.

By the term "amino" is meant a primary (-NH₂), secondary (-NHR), tertiary (-N(R)₂), or quaternary (-N⁺(R)₄) amine, where R is a hydrocarbon chain, a hydroxy, a carbocycle, a heterocycle, or a hydrocarbon substituted with a carbocycle or a heterocycle.

By the term "carboxyl" is meant a free acid -COOH, as well as esters thereof, such as alkyl, aryl, and aralkyl esters. In some embodiments, the esters are methyl, ethyl, propyl, butyl, i-butyl, s-butyl, and t-butyl esters.

By the term "carbocycle" is meant a mono-, bi-, or tri-cyclic carbon ring or ring system having 4-16 members (including bridged members) which is saturated, unsaturated, or partially unsaturated, including aromatic (aryl) ring systems (unless specified as non-aromatic). Non-limiting examples of non-aromatic carbocyclic rings are cyclopropyl, cyclopropentyl, cyclobutyl, cyclobutenyl, cyclopentyl, cyclopentenyl, cyclohexyl, and cyclohexenyl. Non-limiting examples of aromatic carbocyclic rings are phenyl and naphthyl.

By the term "heterocycle" is meant a mono-, bi-, or tri-cyclic ring system having 5-16 members, where at least one ring atom is a heteroatom (i.e., N, O, and S, as well as SO or SO₂). The ring system is saturated, unsaturated, or partially unsaturated and may be aromatic (unless specified as non-aromatic). Non-limiting examples of heterocycles are piperidine, piperazine, pyridine, pyrazine, pyrimidine, pyridazine, morpholine, pyran, pyrole, furan, thiophene (thienyl), imidazole, pyrazole, thiazole, isothiazole, dithiazole, oxazole, isoxazole, dioxazole, thiadiazole, oxadiazole, tetrazole, triazole, thiatriazole, oxatriazole, thiadiazole, and purine and benzofused derivatives thereof.

By the term "hydrocarbon chain" is meant a saturated, unsaturated, linear, or branched carbon chain (i.e., alkyl, alkenyl, or alkynyl). Non-limiting examples of hydrocarbon chains contain 1-12 carbon atoms, 1-6 carbon atoms, or 1-4 carbon atoms (e.g., methyl, ethyl, propyl, butyl, or allyl).

By the phrase "optionally substituted with" is meant to mean, unless otherwise stated, that one or more of the specified substituents is covalently attached to the substituted moiety. When there is more than one substituent, the substituents can be the same or different groups.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure 1 shows non-limiting examples of small molecules of Formula I that can be used in any of the methods described herein.

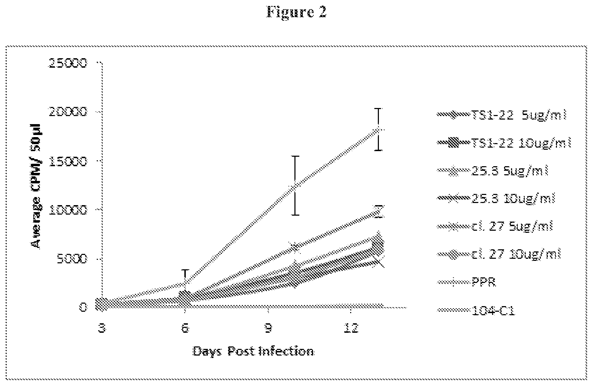

Figure 2 is a graph showing the reverse transcripase activity (counts per minute) in feline 104-C1 T-cells at 3, 6, 9, or 12 days post-infection following a 2-hour spin inoculation with a preparation of FIV-PPR preincubated for 30 minutes with 0 µg/mL (control; PPR), 5 µg/mL, or 10 µg/mL TS1-22 antibody, clone 25.3 antibody, or clone 27 antibody, recovery for 3 hours at 37°C and 5% CO₂, and incubation in fresh media containing the identical concentrations of each antibody for the duration of the study.

### DETAILED DESCRIPTION

Provided herein are methods and compositions for treating or reducing retroviral infections in felines.

### Feline Retroviral Diseases

The feline retroviruses feline immunodeficiency virus (FIV) and feline leukemia virus (FELV) cause disease in felines. FIV is a member of the lentivirus subfamily of retroviruses. It appears to be widely distributed worldwide since the 1960s. There are 5 FIV subtypes (clades A to E) and they all are infection to a variety of susceptible wild and domestic feline species. In the U.S.A., the prevalence of FIV infection for domestic cats is estimated to be 1-4%, and this number is larger in the feral cat population.

FIV has a primary tropism for lymphocytes and gradually destroys subpopulation of T-lymphocytes. This cytopathic effect causes a progressive loss of CD4⁺ T-cells, a decrease in the CD4⁺ T-cell/CD8⁺ T-cell ratio, and the eventual loss of measurable levels of CD8⁺ T-cells in the late stages of infection. Cell-mediated immunity is impaired to a greater extent than antibody-mediated immunity. In addition, impaired production and dysregulation of various cytokines plays a role in pathogenesis of the disease. Antiretroviral drugs are too toxic for felines, leaving virtually no alterative for treating FIV.

FELV is an RNA retrovirus and was described in 1964 (Jarrett et al., Nature 202:566, 1964). FELV and FIV are in the same viral family. Four subgroups of FELV exist: A, B, C, and T. Approximately 0.5% of domestic felines are persistently infected with FELV, but many more domestic felines (>35%) have specific IgG antibodies. Transmission of FELV is mainly through saliva and friendly behaviors, such as sharing feeding bowls and mutual grooming.

FELV causes uncontrolled proliferation of blood cells in felines. Additional symptoms of FELV in felines include infections of the skin, bladder, and respiratory tract, oral disease, seizures, lymphadenopathy, skin lesions, fatigue, fever, weight loss, stomatitis, gingivitis, litter box avoidance, pancytopenia, poor grooming, reoccurring bacterial and viral illnesses, anemia, diarrhea, and jaundice.

Feline retroviral infection can be diagnosed by a skilled veterinary professional (e.g., a veterinarian, a veterinarian assistant, a veterinary technician, or a lab technician). For example, a feline retroviral infection may be determined by the observation of one or more symptoms of a retroviral infection in a feline. Non-limiting examples of symptoms of retroviral infection in a feline include, in addition to those listed above: decreased levels of CD4⁺ T-cells (e.g., relative to a control feline or the same feline prior to infection), decreased ratio of CD4⁺ T-cells to CD8⁺ T-cells (e.g., relative to a control feline or the same feline prior to infection), decreased total white cell count (e.g., relative to a control feline or the same feline prior to infection), depression, lack of apetite, discharge from eyes and nose, fever, still and painful joints and muscles, difficulty breathing, sores in the mouth or on the lips, tongue, feet, or nose, weight loss, diarrhea, enlarged lymph nodes, skin infection, bladder infection, upper respiratory infection, seizures, dementia, vomiting, bloody diarrhea, weakness, conjunctivitis, stomatitis, odontoclasia, periodontitis, gingivitis, rhinitis, pneumonitis, enteritis, and dermatitis. Another symptom of a feline retroviral infection is dementia. A feline retroviral disease can also be diagnosed using commercially available diagnostic kits (e.g., an assay that measures antibody titers in a feline that bind to the retrovirus (e.g., bind to a retroviral protein), an assay that measures the presence of proteins present in the retrovirus, or an assay that measures the presence of a nucleic acid present in the retrovirus). For example, diagnostic kits for the diagnosis of FIV or FELV in a feline are commercially available from Antigen Rapid (FIV Ab/FeLV Ag Test Kit) and Idexx (SNAP® Feline Triple® Test). Another diagnostic kit for the diagnosis of FIV is commercially available from Vita-Tech Laboratories (Feline Immunodeficiency Virus (FIV) DNA Confirmatory Test).

A skilled veterinary professional can determine the effectiveness of treatment of a retroviral infection in a feline by monitoring the number of symptoms or the severity, frequency, and/or duration of one or more symptoms of a retrovirus infection in a feline. For example, a useful treatment may result in a statistically significant or observable decrease in the severity, duration, or frequency of one or more (e.g., one, two, three, four, or five) symptoms in a feline having a retroviral infection. A useful treatment may also result in a decrease of retroviral titers in the feline (e.g., a decrease in retroviral titers in a specific biological fluid of the feline, e.g., serum, blood, lung fluid, nasal fluid, intestinal fluid, or gastic fluid). A useful treatment of FIV or FELV can also result in one or more (e.g., one, two, three, four, or five) of: an increase in CD4⁺ T-cell level (e.g., relative to a control feline having FIV or FELV, but not receiving treatment or the same feline prior to treatment), an increase in the CD4⁺ T-cell to CD8⁺ T-cell ratio (e.g., relative to a control feline having FIV or FELV, but not receiving treatment or the same feline prior to treatment), a decrease in CD8⁺ T-cell-mediated killing of CD4⁺ T-cells (e.g., relative to a control feline having FIV or FELV, but not receiving treatment or the same feline prior to treatment), a decrease retroviral virion or syncytium fusion with non-infected feline cells (e.g., relative to a control feline having FIV or FELV, but not receiving treatment or the same feline prior to treatment), a decrease in syncytium transmission in a feline (e.g., relative to the amount of syncytium transmission in a feline without FIV or FELV, or in the same feline prior to treatment), and a decrease in the development or rate of onset of retrovirus-induced dementia (e.g., relative to a control feline having FIV, but not receiving treatment or the same feline prior to treatment). A skilled veterinary professional may adjust the treatment (e.g., dosage of agents and/or frequency or duration of administration of agents) based on the assessment of the effectiveness of the retroviral infection treatment as described herein.

### LFA-1 and ICAM-1

Lymphocyte function-associated antigen-1 (LFA-1) is a cell surface heterodimer of CD11a and CD18 proteins that is a variety of cells, including T-cells, B-cells, macrophages, and neutrophils. LFA-1 is involved in the recruitment of cells to the site of infection. LFA-1 also binds to ICAM-1 on antigen-presenting cells, and functions as an adhesion molecule. In any of the embodiments described herein, an antibody or an antigen-binding fragment thereof can bind to CD11a or CD18, or may bind to an epitope formed by both CD11a and CD18. In any of the embodiments described herein, at least one small molecule can bind to CD11a or CD18, or both CD11a and CD18.

Intercellular adhesion molecule 1 (ICAM-1), also known as cluster of differentiation 54 (CD54), is a cell surface glycoprotein that is typically expressed on endothelial cells and cells of the immune system (e.g., antigen-presenting cells). As noted above, ICAM-1 binds to LFA-1.

Provided are methods that treat or reduce (e.g., significantly reduce) the likelihood of developing a retroviral infection in a feline. These methods require administering to a feline at least one (e.g., one, two, three, or four) agent (e.g., a small molecule or antibody or antigen-binding fragment thereof) that specifically binds to CD11a and/or CD18 (specifically binds to CD11a, CD18, or an epitope formed by both CD11a and CD18), or ICAM-1, and/or prevents LFA-1 present in a retroviral virion or syncytium from binding to ICAM-1 expressed on the surface of a feline cell (e.g., a non-infected cell). In some embodiments, the at least one agent prevents LFA-1 present in a feline cell (e.g., a non-infected feline cell) from binding to ICAM-1 in a virion or syncytium.

During a retroviral infection in a feline, virions bud off from or are released from an infected host feline cell. Syncytia fuse (transiently or permanently) with non-infected feline cells. Retrovral virions incorporate cellular proteins (e.g., CD11a, CD 18, or ICAM-1) expressed in feline cells during budding from the host cell. A syncytium incorporates cellular proteins (e.g., CD11a, CD18, or ICAM-1) expressed in a feline cell during fusion of the syncytium with a feline cell. CD11a and CD18 present in the virion or syncytium can form LFA-1 (a heterodimer of CD11a and CD18). CD11a, CD18, or ICAM-1 present in the virion or syncytium can aid or facilitate the binding and/or entry of the retroviral virion, or the binding and/or fusion of a syncytium with a non-infected target cell in the feline. In some embodiments, the binding, entry, and/or fusion is facilitated by LFA-1 present on the surface of a virion or syncytium binding to ICAM-1 present on the surface of a non-infected feline cell (e.g., directly or indirectly facilitating retroviral virion binding and/or entry, or syncytium binding and/or fusion with a non-infected feline cell). In some embodiments, the binding, entry, and/or fusion is facilitated by LFA-1 present on the surface of a non-infected feline cell binding to ICAM-1 present on the surface of a virion or syncytium (e.g., directly or indirectly facilitating retroviral virion binding and/or entry, or syncytium binding and/or fusion with a non-infected feline cell).

In any of the methods described herein and used throughout, LFA-1 or ICAM-1 can be found on the surface of a retroviral virion or syncytium or can be found in a feline cell (e.g., a non-infected feline cell).

### Agents

Provided herein are methods of treating or reducing the likelihood of developing a retroviral infection that require the administration of at least one agent that prevents or decreases the binding of LFA-1 to ICAM-1.

Agents useful in the methods described herein include, without limitation, antibodies and antigen-binding fragments thereof, and small molecules. Non-limiting examples of antibodies and antigen-binding fragments thereof and small molecules that prevent or decrease the binding of LFA-1 to ICAM-1 are described herein. Additional agents useful for preventing or decreasing the binding of LFA-1 to ICAM-1 are known in the art. Additional agents useful in the methods described herein can be identified using the screening methods described herein. Two or more (e.g., two, three, four, or five) of the agents described herein can be administered to a feline in any combination without limitation.

### Antibodies and Antigen-Binding Fragments Thereof

Agents useful in any of the methods described herein include antibodies and antigen-binding fragments thereof. In some embodiments, the antibodies and antigen-binding fragments thereof bind to an epitope on CD11a, CD18, or ICAM-1, or an epitope formed by both CDR11a and CD18, that is present in or on the surface of a virion or syncytium or bind to an epitope on CD11a, CDD18, or ICAM-1, or an epitope formed by both CD11a and CD18, that is present in or on the surface of a non-infected feline cell. In some embodiments, the antibodies and antigen-binding fragments thereof bind to an epitope on ICAM-1 (e.g., ICAM-1 present in or on the surface of a virion, syncytium, or non-infected feline cell).

In some embodiments, the antibody or antigen-binding fragment thereof specifically binds to (e.g., binds to an epitope present in) feline CD11a, CD18, ICAM-1, or an epitope formed by both CD11a and CD18. In some embodiments, the antibody or antigen-binding fragment thereof prevents or reduces the binding of a LFA-1 present in or on the surface of a virion or syncytium to ICAM-1 present in or on the surface of a feline cell (e.g.. a non-infected feline cell). In some embodiments, the antibody or antigen-binding fragment thereof prevents or reduces the binding of a LFA-1 present in or on the surface of a non-infected feline cell to ICAM-1 present in or on the surface of a virion, or syncytium. The prevention or reduction of binding of a LFA-1 to ICAM-1 can occur directly (the antibody or antigen-binding fragment thereof binds directly to CD11a, CD18, ICAM-1, or an epitope formed by both CD11a and CD18) or indirectly (the antibody or antigen-binding fragment thereof binds to a protein, lipid, and/or carbohydrate that directly binds to CD11a, CD 18, ICAM-1, or both CD 11 a and CD 18).

Methods for determining the ability of an antibody or antigen-binding fragment thereof to bind to a target protein (e.g., CD11a, CD18, ICAM-1, or an epitope formed by both CD11a and CD18) can be performed using methods known in the art. Non-limiting examples of such methods include competitive binding assays using antibodies known to bind the target protein (e.g., CD11a, CD18, ICAM-1, or an epitope formed by both CD11a and CD18), enzyme-linked immunosorbent assays, BioCoRE®, affinity columns, immunoblotting, or protein array technology. In some embodiments, the binding activity of the antibody or antigen-binding fragment thereof is determined by contacting a feline cell (e.g., a CD8⁺ T-cell, a CD4⁺ T-cell, a dendritic cell, a fibroblast, or an epithelial cell) with the antibody or antigen-binding fragment thereof. Additional methods for identifying agents (antibodies or antigen-binding fragments thereof) that bind to CD11a, CD18, ICAM-1, or an epitope formed by both CD11a and CD18 are described heren.

In some embodiments of any of the methods described herein, the antibody or antigen-binding fragment thereof binds to CDR11a, CD18, ICAM-1, or an epitope formed by both CD11a and CD18 with an K_{D} equal to or less than 1 x 10⁻⁷ M, a K_{D} equal to or less than 1 x 10⁻⁸ M, a K_{D} equal to or less than 5 x 10⁻⁸ M, a K_{D} equal to or less than 1 x 10⁻⁹ M, or a K_{D} equal to or less than 5 x 10⁻⁹ M under physiological conditions (e.g., phosphate buffered saline).

An antibody can be any immunoglobulin or antibody (including, for example, variants, derivatives, and conjugates thereof) that specifically binds an antigen, such as light or heavy chain immunoglobulin molecules and immunologically-active fragments of immunoglobulin molecules. An antibody can also be a single-chain antibody (e.g., a single-domain antibody), such as a single-chain camelid or cartilagenous fish (e.g., shark) antibody, or a single-chain antibody that contains at least one camelid variable antigen-binding domain (VHH) or at least one cartilagenous fish (e.g., shark) immunoglobulin new antigen receptor (Ig-NAR) domain (see, for example, the antibodies described in U.S. Patent Publication No. 2010/0092470). An antibody can be a whole antibody molecule or an antibody multimer.

Antibodies as referred to herein include variants (including derivatives) of antibodies, antibody multimers, and antibody fragments. Examples of antibodies include, but are not limited to: single-chain Fvs (scFvs), single-domain antibodies (e.g., mini-antibodies, micro-antibodies, subnano-antibodies, and nano-antibodies, see for example, the antibodies described in U.S. Patent Application Publication No. 2010/0092470), Fab fragments, Fab' fragments, F(ab')₂, disulfide-linked Fvs (sdFvs), Fvs, and fragments containing, either a VL or a VH domain. The term "single chain Fv" or "scFv" as used herein refers to a polypeptide comprising at least one VL domain of antibody linked to at least one VH domain of an antibody.

Antibodies useful in the methods described herein include, but are not limited to, polyclonal, monoclonal, multispecific (e.g., bi-specific), feline, human, mouse, rabbit, or rat antibodies, chimeric antibodies (e.g., human-feline chimera, mouse-feline chimera, rat-feline chimera, or rabbit-feline chimera), single chain antibodies (e.g., single-domain antibodies), Fab fragments, F(ab') fragments, intracellularly-made antibodies (i.e., intrabodies), epitope-binding fragments of any of the above, and any of the other antibodies or antigen-binding fragments described herein. The antibodies or antigen-binding fragments thereof can be of any type (e.g., IgG, IgE, IgM, IgD, IgA, and IgY), class (e.g., IgG₁, IgG₂, IgG₃, IgG₄, IgA₁, and IgA₂) or subclass. In some embodiments, the antibody or antigen-binding fragment thereof is an IgG₁ antibody or fragment thereof. In other embodiments, the antibody or antigen-binding fragment thereof is an IgG₄ antibody or antigen-binding fragment thereof. Immunoglobulins may have both a heavy and light chain.

An isolated feline CD11a, CD18, ICAM-1, or LFA-1 (heterodimer of CD11a and CD18), or fragment thereof can be used as an immunogen to generate antibodies using standard techniques for polyclonal and monoclonal antibody preparation. For example, feline CD11a can be purified using the methods described in Shimojima et al. (Microbes Infection 5:1171-1176, 2003). In this method, feline CD11a is isolated by first cloning feline CD11a from a periperhal blood mononuclear cell (PBMC) cDNA library using the homologue cloning method using the polymerase chain reaction (Nishimura et al., Immunogenetics 50:369-370, 1999) and expressing the cDNA in insect cells using a recombinant baculovirus vector containing the cloned cDNA downstream of a polyhedrin promoter (Shimojima et al., J. Vet. Med. Sci. 59:467-469, 1997; and Shimojima et al., Vet. Immunol. Immunopathol. 61:17-23, 1998).

The full-length polypeptide or protein (e.g., CD11a, CD18, LFA-1, or ICAM-1) can be used or, alternatively, antigenic peptide fragments can be used as immunogens. The antigenic peptide of a protein comprises at least 8 (e.g., at least 10, 15, 20, or 30) amino acid residues of the amino acid sequence of the protein (e.g., CD11a, CD18, or ICAM-1) and encompasses an epitope of the protein such that an antibody raised against the peptide forms a specific immune complex with the protein.

An immunogen typically is used to prepare antibodies by immunizing a suitable subject (e.g., rabbit, goat, mouse, or other mammal). An appropriate immunogenic preparation can contain, for example, a recombinantly expressed or a chemically synthesized polypeptide. The preparation can further include an adjuvant, such as Freund's complete or incomplete adjuvant, or a similar immunostimulatory agent.

Polyclonal antibodies can be prepared as described above by immunizing a suitable subject with feline CD11a, CD18, ICAM-1, or LFA-1, or an antigenic peptide thereof, as an immunogen. The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. At an appropriate time after immunization, e.g., when the specific antibody titers are highest, antibody-producing cells can be obtained from the subject and used to prepare monoclonal antibodies by standard techniques, such as the hybridoma technique originally described by Kohler et al. (Nature 256:495-497, 1975), the human B cell hybridoma technique (Kozbor et al., Immunol. Today 4:72, 1983), the EBV-hybridoma technique (Cole et al., Monoclonal Antibodies and Cancer Therapy, Alan R. Liss, Inc., pp. 77-96, 1985), or trioma techniques. The technology for producing hybridomas is well known (see, generally, Current Protocols in Immunology, 1994, Coligan et al. (Eds.), John Wiley & Sons, Inc., New York, NY). Hybridoma cells producing a monoclonal antibody are detected by screening the hybridoma culture supernatants for antibodies that bind the polypeptide of interest, e.g., using a standard ELISA assay.

As an alternative to preparing monoclonal antibody-secreting hybridomas, a monoclonal antibody directed against a polypeptide can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP* Phage Display Kit, Catalog No. 240612). Additionally, examples of methods and reagents particularly amenable for use in generating and screening an antibody display library can be found in, for example, U.S. Pat. No. 5,223,409; WO 92/18619; WO 91/17271; WO 92/2079; WO 92/15679; WO 93/01288; WO 92/01047; WO 92/09690; WO 90/02809; Fuchs et al., Bio/Technology 9:1370-1372, 1991; Hay et al., Hum. Antibod. Hybridomas 3:81-85, 1992; Huse et al., Science 246:1275-1281, 1989; Griffiths et al., EMBO J. 12:725-734, 1993.

In some embodiments of any of the methods described herein, the antibodies or antigen-binding fragments are feline antibodies or felinized antibodies. In some embodiments, a felinized antibody is a feline antibody that has been engineered to contain at least one complementary determining region (CDR) present in a non-feline antibody (e.g., a human, rat, mouse, rabbit, or goat antibody). In some embodiments, a felinized antibody or fragment thereof can contain all three CDRs of a light chain of a mouse (e.g., the TS1/22 antibody, the clone 25.3 antibody, or the clone 27 antibody) or human monoclonal antibody that specifically binds to CD11a, CD18, ICAM-1, or an epitope formed by both CD11a and CD18. In some embodiments, the felinized antibody or fragment thereof can contain all three CDRs of a heavy chain of a mouse (e.g., the TS1/22 antibody, the clone 25.3 antibody, or the clone 27 antibody) or human monoclonal antibody that specifically binds to CD11a, CD18, ICAM-1, or an epitope formed by both CD11a and CD18. In some embodiments, the felinized antibody or fragment thereof can contain all three CDRs of a heavy chain and all three CDRs of a light chain of a mouse (e.g., the TS1/22 antibody, the clone 25.3 antibody, or the clone 27 antibody) or human monoclonal antibody that specifically binds to CD11a, CD18, ICAM-1, or an epitope formed by both CD11a and CD18.

Antibodies of the invention may also include multimeric forms of antibodies. For example, antibodies of the invention may take the form of antibody dimers, trimers, or higher-order multimers of monomeric immunoglobulin molecules, Dimers of whole immunoglobulin molecules or of F(ab')₂ fragments are tetravalent, whereas dimers of Fab fragments or scFv molecules are bivalent. Individual monomers within an antibody multimer may be identical or different, i.e., they may be heteromeric or homomeric antibody multimers. For example, individual antibodies within a multimer may have the same or different binding specificities.

Multimerization of antibodies may be accomplished through natural aggregation of antibodies or through chemical or recombinant linking techniques known in the art. For example, some percentage of purified antibody preparations (e.g., purified IgG₁ molecules) spontaneously form protein aggregates containing antibody homodimers and other higher-order antibody multimers. Alternatively, antibody homodimers may be formed through chemical linkage techniques known in the art. For example, heterobifunctional crosslinking agents including, but not limited to, SMCC (succinimidyl 4-(maleimidomethy1)cyclohexane-1-carboxylate) and SATA (N-succinimidyl S-acethylthio-acetate) (available, for example, from Pierce Biotechnology, Inc. (Rockford, IL)) can be used to form antibody multimers. An exemplary protocol for the formation of antibody homodimers is given in Ghetie et al. (Proc. Natl. Acad. Sci. U.S.A. 94: 7509-7514, 1997). Antibody homodimers can be converted to Fab'₂ homodimers through digestion with pepsin. Another way to form antibody homodimers is through the use of the autophilic T15 peptide described in Zhao et al. (J. Immunol. 25:396-404, 2002).

Alternatively, antibodies can be made to multimerize through recombinant DNA techniques. IgM and IgA naturally form antibody multimers through the interaction with the mature J chain polypeptide. Non-IgA or non-IgM molecules, such as IgG molecules, can be engineered to contain the J chain interaction domain of IgA or IgM, thereby conferring the ability to form higher order multimers on the non-IgA or non-IgM molecules (see, for example, Chintalacharuvu et al., Clin. Immunol. 101:21-31, 2001, and Frigerio et al., Plant Physiol. 123:1483-1494, 2000). IgA dimers are naturally secreted into the lumen of mucosa-lined organs. This secretion is mediated through interaction of the J chain with the polymeric IgA receptor (pIgR) on epithelial cells. If secretion of an IgA form of an antibody (or of an antibody engineered to contain a J chain interaction domain) is not desired, it can be greatly reduced by expressing the antibody molecule in association with a mutant J chain that does not interact well with pIgR (Johansen et al., J. Immunol., 167:5185-192, 2001). ScFv dimers can also be formed through recombinant techniques known in the art; an example of the construction of scFv dimers is given in Goel et al. (Cancer Res. 60:6964-71, 2000), Antibody multimers may be purified using any suitable method known in the art, including, but not limited to, size exclusion chromatography.

Any of the antibodies or antigen-binding fragments described herein may be conjugated to a stabilizing molecule (e.g., a molecule that increases the half-life of the antibody or antigen-binding fragment thereof in a feline or in solution). Non-limiting examples of stabilizing molecules include: a polymer (e.g., a polyethylene glycol) or a protein (e.g., serum albumin, such as feline serum albumin). Any of the antibodies or antigen-binding fragments described herein may be conjugated to a label (e.g., a fluorophore) or a therapeutic agent (e.g., a proteinaceous therapeutic agent).

An exemplary anti-CD11a antibody or antigen-binding fragments thereof that can be used in any of the methods described herein is the antibody produced from clone 25.3 (Becker Coulter (PN IMO0157 and PN IM1433U) and antigen-binding fragments of the antibody produced from clone 25.3, the antibody produced from clone 27 (Fisher Scientific No. 610826) and antigen-binding fragments of the antibody produced from clone 27, and TS1/22 and antigen-binding (feline CD11a-binding) fragments of TS1/22. The antibody produced from clone 25.3 has been described as having the ability to bind to an epitope on feline CD11a (Saalmüller et al., Cell. Immunol. 236:51-58, 2005). The antibody produced from clone 25.3 and the TS1/22 antibody have been shown to bind to the same epitope (IdeA) within the I domain of CD11a (Champe et al., J. Biol. Chem. 270:1388-1394, 1995), thus both the antibody produced by clone 25.3 and the TS1/22 antibody (and antigen-binding fragments of each antibody) can be used to perform the present methods. The data provided herein indicate that the antibody produced by clone 27 binds to CD11a, and prevents FIV infection of feline cells (Figure 2).

Additional anti-CD11a antibodies are known in the art and include, for example antibodies produced from clone 2D7, HI111, M17/4, TS2/4, WT.1, or H155-78 (BioLegend, San Diego, CA), efalizumab, the antibody produced from clone MEM-25 (Antibodies Online, Aachen, Germany), and the anti-feline CD11a antibody described in Shimojima et al. (Microbes and Infection 5:1171-1176, 2003), or antigen-binding fragments thereof. Additional anti-CD1a antibodies can contain at least one CDR (e.g., at least three light chain CDRs and/or at least three heavy chain CDRs) of the antibody produced from clone 25.3 (Becker Coulter (PN IM0157 and PN IM1433U), the antibody producted from clone 27 (Fisher Scientific No. 610826), or the anti-feline CD11a antibody described in Shimojima et al. (Microbes and Infection 5:1171-1176, 2003).

Additional antibodies or antigen-binding fragments useful in any of the methods described herein bind competitively with the antibody TS1/22 to feline CD11a (e.g., binds competitively with the antibody TS1/22 to a cell that expresses feline CD11a). Additional antibodies or antigen-binding fragments useful in any of the methods described herein bind competitively with the clone 25.3 antibody to feline CD11a (e.g., binds competitively with the clone 25.3 antibody to a cell that expresses feline CD11a) or bind competitively with the clone 27 antibody to feline CD11a (e.g., binds competitively with the clone 27 antibody to a cell that expresses feline CDR11a). Additional anti-CD11a antibodies are known in the art.

Non-limiting examples of anti-CD18 antibodies or antigen-binding fragments thereof that can be used in any of the methods described herein include antibodies produced from clone M18/2, TS1/18, or WT.3 (BioLegend, San Diego, CA), the antibodies described in U.S. Patent Application Publication No. 2004/0101527, and the antibodies described in U.S. Patent Nos. 6,689,869 and 5,914,112, or antigen-binding fragments thereof. Additional anti-CD18 antibodies are known in the art.

Non-limiting examples of anti-ICAM-1 antibodies or antigen-binding fragments thereof that can be used in any of the methods described herein include antibody #4915 (Cell Signaling Technology), sc-107 (Santa Cruz Biotechnology), RabMab® (Epitomics), anti-CD54 (Cell Applications, Inc.), the antibodies described in U.S. Patent Application Publication No. 2011/0052601, and the antibodies described in U.S. Patent No. 5,324,510, 5,695,760, or antigen-binding fragments thereof. Additional anti-ICAM-1 antibodies are known in the art.

### Small molecules

Agents useful in the methods described herein also include small molecules. Small molecules useful in the methods described herein can be small organic (e.g., peptides, nucleotides, sugars, and/or lipids) or small inorganic molecules (e.g., metal complexes). Small molecules useful in the described methods may be identified using any of the screening methods described herein. Small molecules can block or decrease binding of LFA-1 to ICAM-1 by binding directly to CD11a, CD18, ICAM-1, or both CDR11a and CD18. In some embodiments, the small molecule can bind LFA-1 at or around the same site that ICAM-1 binds to LFA1-1. In some embodiments, the small molecule can bind LFA-1 at a site that is remote from the site that ICAM-1 binds to LFA-1 (e.g., the binding of the small molecule induces a change in the tertiary structure of LFA-1 that prevents or decreases binding of LFA-1 to ICAM-1, or the binding of the small molecule prevents the oligomerization or heterodimerization of LFA-1 that prevents or decreases the the binding of LFA-1 to ICAM-1).

In some embodiments, the small molecule can bind ICAM-1 at or around the same site that LFA-1 binds to ICAM-1. In some embodiments, the small molecule can bind ICAM-1 at a site that is remote from the site that LFA-1 binds to ICAM-1 (e.g., the binding of the small molecule induces a change in the tertiary structure of ICAM-1 that prevents or decreases binding of ICAM-1 to a LFA-1, or the binding of the small molecule prevents the oligomerization of ICAM-1 that prevents or decreases the the binding of ICAM-1 to LFA-1).

In some embodiments, the small molecules can block or decrease binding of LFA-1 to ICAM-1 by binding to another molecule (e.g., protein, lipid, and/or carbohydrate) that binds to CD11a, CD18, ICAM-1, or both CD11a and CD18.

Non-limiting examples of small molecules that can be used in any of the methods described herein include statin or statin derivatives. Lovastatin and simvastatin have been demonstrated to inhibit LFA-1 activation (Wang et al., Biol. Blood Marrow Transplant. 15:1513-1522, 2009; Almog, Chest 124:740-743, 2003). Some statin derivatives (e.g., LFA878, LFA703, LFA451, and XVA143) have been identified as preventing the ability of CD11a to bind to ICAM-1 (Weitz-Schmidt et al., J. Biol. Chem. 279:46764-46771, 2004: Welzenbach et al., J. Biol. Chem. 277:10590-10598, 2002).

Thus, lovastatin, simvastatin, lovastatin derivatives, simvastatin derivatives, LFA878, LFA703, LFA451, and XVA143, as well as other statins may prevent or decrease the ability of LFA-1 (comprising CD11a and CD18) from binding to ICAM-1.

Non-limiting examples of small molecules that can be used in any of the methods described herein include,: lovastatin, simvastatin, pravastatin, atorvastatin, fluvastatin, rosuvastatin, amplodipine, cerivastatin, mevastatin, pitavastatin, lovastatin, derivatives, simvastatin derivatives, LFA878, LFA703, LFA451, and XVA143. In some embodiments of any of the methods described herein the small molecule is lovastatin, simvastatin, a lovastatin derivative, a simvastatin derivative, LFA878, LFA1703, LFA1451, and XVA143, or a salt, solvate, or hydrate thereof.

Lovastatin has the following chemical structure:

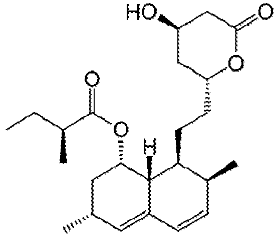

Derivatives of lovastatin can be generated using methods of chemical modification known in the art. For example, methods of generating derivatives of lovastatin, as well as lovastatin derivatives (e.g., exomethylene-modified, an 8-acyl-modified, or an alkylated lovastatin derivatives) are described in U.S. Patent Nos. 4,866,186 and 6,472,542).

Simvastatin has the following chemical structure:

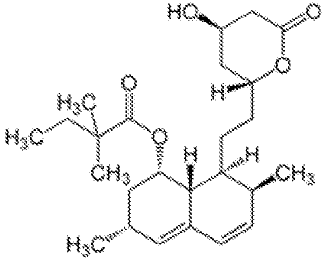

Derivatives of simvastatin can be generated using methods of chemical modification known in the art. For example, methods of generating derivatives of simvastatin, as well as simvastatin derivatives (e.g., 3-keto, 5-hydroxy-derivatives, di-and hydroxy derivatives) are described in U.S. Patent Nos. 6,252,091; 6,541,511: 6,384,238; 6,252,091; 6,100,407; 5,393,893; 5,159,104; and 4,965,200; and in U.S. Patent Application Publication Nos. 2003/0176501 and 2002/0035274.

Additional methods for the modification of statins (e.g., lovastatin and simvastatin) are described in U.S. Patent Nos.5,134,124; 7,855,302; 5,462,716; 7,563,909; 7,420,078; 7,304,091; 7,297,808; and 7,166,638; and U.S. Patent Application Publication Nos. 2011/0054193; 2009/0118317; 2008/0289056; 2008/0096908; 2008/0090857; 2007/0072942; 2005/0228042; 2005/0165084; 2005/0148654; 2004/0235935; and 2004/0186313.

Statin derivatives (e.g., lovastatin and simvastatin derivatives) can be used in any of the methods described herein. The ability of a statin derivative, a simvastatin derivative, or a lovastatin derivate to decrease LFA-1 binding to ICAM-1 can be determinined using affinity assays known in the art (e.g., BioCore technology) and assays described herein.

Additional small molecules that are useful in any of the methods described have the structure of Formula I:

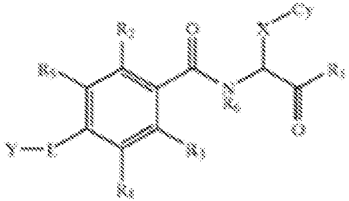

In Formula I, Cy is a non-aromatic carbocycle or heterocycle, optionally substituted with hydroxy (-OH), mercapto (-SH), thioalkyl, a halogen (e.g., F, Cl, Br, or I), oxo (=O), thio (=S), amino, aminoalkyl, amidine (-C(NH)-NH₂), guanidine (-NH₂-C(NH)-NH₂), nitro, alkyl, or alkoxy. In some embodiments, Cy is a 3-5 member ring. In some embodiments, Cy is a 5- or 6-member non-aromatic heterocycle, optionally substituted with hydroxy, mercapto, halogen (e.g., F or Cl), oxo (=O), thio (=S), amino, amidine, guanidine, nitro, alkyl, or alkoxy. In some embodiments, Cy is a 5-member non-aromatic heterocycle, optionally substituted with hydroxy, oxo, thio, Cl, C₁₋₄ alkyl (e.g., methyl), or C₁₋₄ alkanoyl (e.g., acetyl, propanoyl, or butanoyl). In some embodiments, the non-aromatic heterocycle contains one or heteroatoms (N, O, or S) and is optionally substituted with hydroxyl, oxo, mercapto, thio, methyl, acetyl, propanoyl, or butyl. In some embodiments, the non-aromatic heterocycle contains at least one nitrogen atom that is optionally substituted with methyl or acetyl. In some embodiments, the non-aromatic heterocycle is selected from: piperidine, piperazine, morpholine, tetrahydrofuran, tetrabydrothiophene, oxazolidine, and thiazolidine, optionally substituted with hydroxy, oxo, mercapto, thio, alkyl, or alkanoyl. In some embodiments, Cy is a non-aromatic heterocycle selected from: tetrahydrofuran-2-yl, thiazolidin-5-yl, thiazolidin-2-one-5-yl, thiazolidin-2-thione-5-yl, and cyclopropapyrrolidine.

In some embodiments Cy is a 3-6 member carbocycle, optionally substituted with hydroxyl, mercapto, halogen, oxo, thio, amino, amidine, guanidine, alkyl, alkoxy, or acyl. In some embodiments, the carbocycle is saturated or partially unsaturated. In some embodiments Cy is a carbocycle selected from the group consisting of cyclopropyl, cyclopropenyl, cyclobutyl, cyclobutenyl, cyclopentyl, cyclopentenyl, cyclohexyl, and cyclohexenyl.

In Formula I, X is a C₁₋₅ divalent hydrocarbon linker, optionally having one or more carbon atoms replaced with N, O, S, SO, or SO₂, and is optionally substituted with hydroxyl, mercapto, halogen, amino, aminoalkyl, nitro, oxo, or thio. In some embodiments X will have at least one carbon atom. Replacements and substitutions can form an amide moiety (-NRC(O)- or -C(O)NR-) within the hydrocarbon chain or at either or both ends. Other moieties can include sulfonamide (-NRSO₂- or -SO₂NR), acyl, ether, thioether, and amine. In some embodiments, X is the group -CH₂-NR₆-C(O)-, where the carbonyl -C(O)- portion thereof is adjacent (i.e., covalently bonded) to Cy and R₆ is alkyl (e.g., methyl) or H.

In Formula I, Y is a carbocycle or heterocycle, optionally substituted with hydroxyl, mercapto, halogen, oxo, thio, a hydrocarbon, a halo-substituted hydrocarbon, amino, amidine, guanidine, cyano, nitro, alkoxy, or acyl. In some embodiments, Y is aryl or heteroaryl, optionally substituted with halogen or hydroxyl. In some embodiments, Y is phenyl, furan-2-yl, thiophene-2-yl, phenyl substituted with a halogen (e.g., Cl) or hydroxyl (e.g., at the meta position).

In Formula I, L is a divalent hydrocarbon, optionally having one or more carbon atoms replaced with N, O, S, SO, or SO₂, and optionally being substituted with hydroxyl, halogen, oxo, or thio; or three carbon atoms of the hydrocarbon are replaced with an amino acid residue. In some embodiments, L is less than 10 atoms or less than 5 atoms in length. In some embodiments, L is 5 or 3 atoms in length. In some embodiments, L is selected from: -CH=CH-C(O)-NR₆-CH₂-, -CH₂-NR₆-C(O)-, -C(O)-NR₆-CH₂-, -CH(OH)-(CH₂)₂-, -(CH₂)₂-CH(OH)-, -(CH₂)₃-, -C(O)-NR₆-CH(R₇)-C(O)-NR₆-, -NR₆-C(O)-CH(R₇)NR₆-C(O)-, -CH(OH)-CH₂-O-, and -CH(OH)-CF₂-CH₂-, where each R₆ is independently H or alkyl, and R₇ is an amino acid side chain. Amino acid side chains can include non-naturally occurring side chains, such as phenyl, or naturally-occurring side chains. In some embodiments the side chains are Phe, Tyr, Ala, Gln, and Asn. In some embodiments, L is -CH=CH--C(O)-NR₆-CH₂-, where the-CH=CH- moiety is adjacent (i.e., covalently bounded) to Y. In some embodiments, L is -CH₂-NR₆-C(O)-, where the methylene moiety (-CH₂-) is adjacent to Y.

In Formula I, R₁ is H, OH, amino, O-carbocycle, or alkoxy, optionally substituted with amino, a carbocycle or a heterocycle. In some embodiments, R₁ is H, phenyl, or C₁₋₄ alkoxy, optionally substituted with a carbocycle such as phenyl. In some embodiments, R₁ is H. In some embodiments, R₁ is methoxy, ethoxy, propyloxy, butyloxy, isobutyloxy, s-butyloxy, t-butyloxy, phenoxy, or benzyloxy. In some embodiments R₁ is NH₂. In some embodiments, R₁ is ethoxy. In some embodiments, R₁ is isobutyloxy. In some embodiments, R₁ is an alkoxy substituted with amino, for example, 2-aminoethoxy, N-morpholinoethoxy, N,N-dialkyaminoethoxy, or quaternary ammonium hydroxy alkoxy (e.g., trimethylammoniumhydroxyethoxy).

In Formula I, R₂₋₅ are independently H, hydroxyl, mercapto, halogen, cyano, amino, amidine, guanidine, nitro, or alkoxy; or R₃ and R₄ together form a fused carbocycle or heterocycle, optionally substituted with hydroxyl, halogen, oxo, thio, amino, amidine, guanidine, or alkoxy. In some embodiments, R₂ and R₃ are independently H, F, Cl, Br, or I. In some embodiments, R₄ and R₅ are both H. In some embodiments, one of R₂ and R₃ is a halogen, while the other is hydrogen or a halogen. In some embodiments, R₃ is Cl, while R₂, R₄, and R₅ are each H. In some embodiments, R₂ and R₃ are both C1, while R₄ and R₅ are both H.

R₆ is H or a hydrocarbon chain, optionally substituted with a carbocycle or a heterocycle. In some embodiments, R₆ is H or alkyl (e.g., methyl, ethyl, propyl, butyl, i-butyl, s-butyl, or t-butyl). In some embodiments R₆ is H. Non-limiting specific examples of compounds of Formula I are shown in Figure 1. Additional examples of small molecules of Formula I are described in U.S. Patent No. 6,872,735.

Small molecules of Formula I can be prepared according to established organic synthesis techniques from starting materials and reagents that are commercially available. Many standard chemical techniques and procedures are described in March, J., "Advanced Organic Chemistry," McGraw-Hill, New York, 1977; and Collman, J., "Principles and Applications of Organotransition Metal Chemistry," University Science, Mill Valley, 1987; and Larock, R., "Comprehensive Organic Transformations," Verlag, New York, 1989. Depending on the particular substituents present on the compounds, suitable protection and deprotection procedures may be required. Numerous protecting groups are described in Greene and Wuts, "Protective Groups in Organic Chemistry," 2nd Edition, John Wiley and Sons, 1991, as well as detailed
protection and deprotection procedures. For example, suitable amino protecting groups include t-butyloxycarbonyl (Boc), fluorenylmethylcarbonyl (Fmoc), 2-trimethylsilylethyoxycarbonyl (Teoc), 1-methyl-1-(4-biphenylyl)ethoxycarbonyl (Bpoc), allyloxycarbonyl) (Alloc), and benzyloxycarbonyl (Cbz). Carboxyl groups can be protected as fluorenylmethyl groups or alkyl esters (e.g., methyl, ethyl, or alkenyl esters, such as allyl). Hydroxyl groups can be protected with trityl, monomethoxytrityl, dimethoxytrityl, and trimethoxytrityl groups. Additional specific methods for generating small molecules of Formula I are described in U.S. Patent No. 6,872,735.

### Veterinary Compositions

Also provided herein are veterinary compositions that contain at least one (e.g., one, two, three, four, or five) of the agents described herein. In some embodiments, the at least one agent decreases (e.g., significantly decreases) or inhibits binding of a LFA-1 to ICAM-1 in a feline.

Two or more (e.g., two, three, four or five) agents can be present in a veterinary composition in any combination, e.g., two or more proteins (e.g., antibodies and/or antigen-binding antibody fragments), two or more small molecules (e.g., lovastatin, simvastatin, lovastatin derivatives, simvastatin derivatives, LFA703, LFA451, LFA878, XVA143, or any compound of Formula I, or any combination thereof), or combinations of at least one protein (e.g., antibodies and/or antigen-binding antibody fragments) and at least one small molecule (e.g., lovastatin, simvastatin, lovastatin derivatives, simvastatin derivatives, LFA703, LFA451, LFA878, XVA143, or any compound of Formula I, or any combination thereof).

The veterinary compositions may be formulated in any manner known in the art. Veterinary compositions are formulated to be compatible with their intended route of administration, whether oral or parenteral (e.g., intravenous, intradermal, subcutaneous, intraperitoneal, transmucosal, or transdermal (e.g., topical ointments, salves, gels, patches or creams as generally known in the art)). The compositions can include a sterile diluent (e.g., sterile water or saline), a fixed oil, polyethylene glycol, glycerine, propylene glycol or other synthetic solvents; antibacterial or antifungal agents such as benzyl alcohol or methyl parabens, chlorobutanol, phenol, ascorbic acid, thimerosal, and the like; antioxidants such as ascorbic acid or sodium bisulfite;
chelating agents such as ethylenediaminetetraacetic acid; buffers such as acetates, citrates or phosphates; and isotonic agents such as sugars (e.g., dextrose), polyalcohols (e.g., manitol or sorbitol), or salts (e.g., sodium chloride). Liposomal suspensions (including liposomes targeted to affected cells with monoclonal antibodies specific for the target feline cell) can also be used as pharmaceutically acceptable carriers (see, e.g., U.S. Patent No. 4,522,811). Preparations of the compositions can be formulated and enclosed in ampules, disposable syringes, or multiple dose vials. Where required (as in, for example, injectable formulations), proper fluidity can be maintained by, for example, the use of a coating such as lecithin, or a surfactant. Absorption of the active ingredient can be prolonged by including an agent that delays absorption (e.g., aluminium monostearate and gelatin). Alternatively, controlled release can be achieved by implants and microencapsulated delivery systems, which can include biodegradable, biocompatible polymers (e.g., ethylene vinyl acetate, polyanhydrides, polyglycolic acid, collagen, polyorthoesters, and polylactic acid; Alza Corporation and Nova Pharmaceutical, Inc.).

Where oral administration is intended, the agent can be included in pills, capsules, troches, and the like, and can contain any of the following ingredients, or compounds of a similar nature: a binder, such as microcrystalline cellulose, gum tragacanth or gelatin; an excipient, such as starch or lactose, a disintegrating agent, such as alginic acid, Primogel, or corn starch; a lubricant, such as magnesium stearate; a glidant, such as colloidal silicon dioxide; a sweetening agent, such as sucrose or saccharin; or a flavoring agent, such as methyl salicylate or fish flavoring.

Compositions containing one or more of any of the agents described herein can be formulated for oral or parenteral administration in dosage unit form (i.e., physically discrete units containing a predetermined quantity of active compound for ease of administration and uniformity of dosage). In some embodiments, one or more agents can be administered to the feline as a component of a food composition (e.g., a pellet, powder, or semi-sold slurry) or liquid (e.g., a syrup) for oral ingestion.

Toxicity and therapeutic efficacy of compositions can be determined by standard pharmaceutical procedures in cell cultures or experimental animals (e.g., felines). One can, for example, determine the LD50 (the dose lethal to 50% of the population) and the ED50 (the dose therapeutically effective in 50% of the population), the therapeutic index being the ratio of LD50:ED50. Agents that exhibit high therapeutic indices are preferred. Where an agent exhibits an undesirable side effect, care should be taken to target that agent to the site of the affected tissue (the aim being to minimize potential damage to unaffected cells and, thereby, reduce side effects). Toxicity and therapeutic efficacy can be determined by other standard pharmaceutical procedures.

Data obtained from cell culture assays and animal studies can be used in formulating an appropriate dosage of any given agent for use in felines. A therapeutically effective amount of the one or more agent will be an amount that treats or decreases the risk of developing a retroviral infection in a feline, decreases the severity, frequency, and/or duration of one or more symptoms of a retroviral infection in a feline, increases the ratio of CD4⁺ T-cells to CD8⁺ T-cells in a feline with a retroviral infection, increases the number of CD4⁺ T cells in a feline with a retroviral infection, and/or increases the total white blood cell count in a feline with a retroviral infection (e.g., as compared to a control feline having a retroviral infection or the same feline prior to treatment). The effectiveness and dosing of any of the agents described herein can be determined by a veterinary professional using methods known in the art, as well as by the observation of one or more symptoms of retroviral infection in a feline. Certain factors may influence the dosage and timing required to affectively treat a feline (e.g., the severity of the infection or disease, previous treatments, the general health and/or age of the feline, and the presence of other diseases).

As noted herein, agents administered according to the methods described herein can be small molecules (e.g., peptides, peptidomimetics (e.g., peptoids), amino acid residues (or analogs thereof), polynucleotides (or analogs thereof), nucleotides (or analogs thereof), or organic or inorganic compounds (e.g., heteroorganic or organometallic compounds)). Typically, such molecules will have a molecular weight less than about 10,000 grams per mole (e.g., less than about 7,500, 5,000, 2,500, 1,000, or 500 grams per more). Salts, esters, and other pharmaceutically acceptable forms of any of these compounds can be assayed and, if a desirable activity is detected, administered according to the therapeutic methods described herein. The agents administered according to the methods described herein can be proteins (e.g., antibodies or antigen-binding fragments thereof).

Exemplary doses include milligram or microgram amounts of any of the agents described herein per kilogram of the feline's weight (e.g., about 1 µg - 500 mg/kg; about 100 µg - 500 mg/kg; about 100 µg - 50 mg/kg; 10 µg - 5 mg/kg; 10 µg - 0.5 mg/kg; or 1 µg - 50 µg/kg). While these doses cover a broad range, one of ordinary skill in the art will understand that therapeutic agents, including small molecules, vary in their potency, and effective amounts can be determined by methods known in the art. Typically, relatively low doses are administered at first, and the attending veterinary professional (in the case of therapeutic application) or a researcher (when still working at the development stage) can subsequently and gradually increase the dose until an appropriate response is obtained. In addition, it is understood that the specific dose level for any particular feline will depend upon a variety of factors including the activity of the specific compound employed, the age, body weight, general health, gender, and diet of the feline, the time of administration, the route of administration, the rate of excretion, any drug combination, and the degree of activity to be modulated.

The veterinary compositions can be included in a container, pack, or dispenser together with instructions for administration.

### Methods of Treating Retroviral Infection in a Feline

Provided herein are methods for treating or reducing the likelihood of deveping a retroviral infection in a feline. Also provided are methods for reducing retroviral virion entry (e.g., fusion) into a feline target cell (e.g., a non-infected cell), retroviral virion budding from a feline cell, and syncytium transmission in a feline. All of the methods described herein require administering to the feline at least one agent that prevents or reduces the binding of LFA-1 (e.g., present in or on the surface of a non-infected feline cell, a virion, or a syncytium) from binding to ICAM-1 (e.g., present in a non-infected feline cell, a virion or a syncytium). In some embodiments, the at least one agent specifically binds to CD11a, CD18, ICAM-1, or an epitope formed by both CAD 11a and CD 18 (e.g., present in or on the surface of a non-infected target cell and/or present in or on the surface of a virion and/or syncytium). In some embodiments, the at least one agent binds directly to CD11a, CD18, ICAM-1, or an epitope formed by both CD11a and CDR18. In some embodiments, the at least one agent indirectly binds to CD11a, CD18, ICAM-1, or an epitope formed by both CD11a and CD18. In any of the methods described herein, the at least one agent can be formulated or administered as one or more of the veterinary compositions described herein.

In some embodiments, the feline is a domestic feline. In some embodiments, the feline that is treated can be previously diagnosed as having a retroviral infection (e.g., FIV or FELV). A feline can be diagnosed as having a retroviral infection by a veterinary professional using any of the methods described herein (e.g., by the observation of at least one symptom of a retroviral infection in a feline) or any methods known in the art. In some embodiments, the feline that is treated can be identified as having an increased risk of developing a retroviral infection (e.g., FIV and FELV). For example, a feline can be determined to have an increased risk of retroviral infection in view of a local pandemic of retroviral infection or by its proximity to one or more other felines having or suspected of having a retroviral infection. A determination or prediction of the incidence of retroviral infection in a population of felines can be assessed or determined by public health officials. In any of the methods described herein, the feline may be treated by its owner (e.g., a domestic feline), a veterinary professional, or a public health worker.

In some embodiments, the agent is an antibody or an antigen-binding fragment thereof that binds specifically to CD11a, CD18, ICAM-1, or an epitope formed by both CD11a and CD18. In some embodiments, the antibody or an antigen-binding fragment thereof prevents or reduces LFA-1 from binding to ICAM-1. Any of the antibodies or antibodies fragments described herein can be used alone or in any combination in any of the methods described herein. In a non-limiting example, the at least one agent used in any of the methods described herein can be a monoclonal antibody, a chimeric antibody, a single-chain antibody (e.g., a single-domain antibody), a feline antibody, or a felinized antibody.

In some embodiments of any of the methods described herein, the at least one agent can be any of the small molecules described herein. In a non-limiting example, the small molecule can be a statin or a statin derivative (e.g., any of the satins or statin derivates described herein, such as lovastatin, simvastatin, a lovastatin derivative (6-exomethylene-modified, an 8-acyl-modified, or an alkylated lovastatin), and/or a simvastatin derivative). Additional small molecules that can be used in any of the methods described herein include LFA703, LFA451, LFA878, and XVA143. Additional small molecules that can be used in any of the methods described herein include molecules of Formula I (as described herein). Non-limiting examples of small molecules of Formula I that can be used in any of the methods described herein are shown in Figure 1. Additional examples of small molecules of Formula I that can be used in any of the methods described herein are listed in U.S. Patent No. 6,872,735.

In some embodiments of any of the methods described herein, the methods include administering to a feline at least one small molecule that prevents or decreases LFA-1 binding to ICAM-1 and/or at least one antibody or antigen-binding fragment thereof that specifically binds to CD11a, CD18, ICAM-1, or an epitope formed by both CD11a and CD18, where the at least one small molecule or the at least one antibody or antigen-binding fragment thereof is administered in an amount sufficient to treat a retrovirus infection, reduce the entry (e.g., fusion) of a retroviral virion into a feline cell, reduce the budding of retroviral virions from a feline cell, and/or reduce syncytium transmission in a feline. The effect of a small molecule and/or an antibody or antigen-binding fragment thereof (as described herein) on virion fusion and/or budding from a feline cell, as well as syncytium transmission can be studied in vitro (in tissue culture) using, for example, microscopic techniques. The data from these in vitro studies can be used to predict the effect of the small molecule and/or antibody or antigen-binding fragment thereof (as described herein) on syncytium transmission in a feline. A decrease in syncytium transmission can also result in a decrease in or a delay in the onset of dementia in a feline having a retroviral (FIV) infection. Feline dementia can be diagnosed by a veterinary care professional or by a feline's owner (for a domestic feline) by the observation of specific behaviors by the feline (e.g., the feline seems to get lost or confused in familiar surroundings, inability or difficulty in finding a litter box (for a domestic feline), or a decrease in the response to an owner's vocal greeting or command).

In some embodiments, the feline is administered at least one antibody or antigen-binding fragment that specifically binds to CD11a, CD18, ICAM-1, or an epitope formed by both CD11a and CD18 (e.g., present in or on the surface of a non-infected feline cell or present in or on the surface of a virion or syncytium). In some embodiments of any of the methods described herein, the feline is administered at least one antibody or antigen-binding fragment thereof that prevents LFA-1 from binding to ICAM-1.

In some embodiments, the feline can be administered at least one antibody or antigen-binding fragment thereof that contains at least one complementary determining region (CDR) from the light chain or the heavy chain of TS1/22 antibody, the clone 25.3 antibody, or the clone 27 antibody. In some embodiments, the feline can be administered at least one antibody or antigen-binding fragment thereof that contains the three CDRs from the light chain of TS1/22 antibody, the clone 25.3 antibody, or the clone 27 antibody or the three CDRs from the heavy chain of TS1/22 antibody, the clone 25.3 antibody, or the clone 27 antibody. In some embodiments, the feline can be administered an antibody or an antigen-binding fragment thereof that contains the three CDRs from the light chain of TS1/22 antibody and the three CDRs from the heavy chain of TS1/22 antibody. In some embodiments, the feline can be administered an antibody or an antigen-binding fragment thereof that contains the three CDRs from the light chain of the clone 25.3 antibody and the three CDRs from the heavy chain of the clone 25.3 antibody. In some embodiments, the feline can be administered an antibody or an antigen-binding fragment thereof that contains the three CDRS from the light chain of the clone 27 antibody and the three CDRs from the heavy chain of the clone 27 antibody.

In some embodiments of any of the methods described herein, the at least one antibody can be a feline antibody or an antigen-binding fragment thereof, or a felinized antibody or an antigen-binding fragment thereof. In some embodiments of any of the methods described herein, the antibody can be a single-chain antibody (e.g., a single-domain antibody). In some embodiments of any of the methods described herein, the at least one antigen-binding fragment thereof can be a Fab fragment, a F(ab')2 fragment, or a scFv fragment.

In some embodiments of any of the methods described herein, the feline is administered at least one (e.g., at least 1, 2, 3, 4, 5, 6, 7, 8, 9, 10, 15, 20, 25, or 30) dose of a composition containing at least one of any of the agents described herein (e.g., administered at least one dose of any of the veterinary compositions described herein). In any of the methods described herein, at least one agent (e.g., any of the agents described herein) or at least one veterinary composition (e.g., any of the veterinary compositions described herein) can be administered intracenously, intaarterially, ocularly, orally, subcutaneously, intraperitoneally, or intramuscularly to the feline. In some embodiments, the at least one agent (e.g., small molecule) or at least one veterinary composition containing a small molecule is administered orally. In some embodiments, at least one antibody or antigen-binding fragment thereof or at least one veterinary composition containing an antibody or antigen-binding fragment thereof is administered intravenously. In some embodiments of any of the methods described herein, a feline is administered at least one agent that is a small molecule (e.g., a small molecule that prevents or reduces LFA-1 from binding to ICAM-1, e.g., any of the small molecules described herein) or at least one veterinary composition that contains a small molecule, and at least one antibody or antigen-binding fragment thereof (e.g., an antibody or antigen-binding fragment thereof that specifically binds to CD11a, CD18, ICAM-1, or an epitope formed by both CD11a and CD18) or at least one veterinary composition that contains an antibody or antigen-binding fragment thereof (e.g., an antibody or antigen-binding fragment thereof that specifically binds to CD11a, CD18, ICAM-1, or an epitope formed by both CD11a and CD18). In some embodiments, at least one small molecule agent and at least one antibody or antigen-binding fragment thereof are administered in the same composition (e.g., the same veterinary composition). In some embodiments, at least one small molecule and at least one antibody or antigen-binding fragment thereof are administered to the feline using different routes of administration.

In some embodiments, the administering of at least one of any of the agents or at least one of any of the veterminary compositions described herein results in a decrease in the severity, frequency, or duration of at least one symptom of a retroviral infection in the feline. In some embodiments, the administering of at least one of any of the agents or at least one of any of the veterinary compositions described herein results in a decrease in retroviral titer in the feline. In some embodiments, the administering of at least one agent or at least one veterinary composition as described herein results in an increase (e.g., a significant increase in the ratio of CD4⁺ T-cells to CD8⁺ T-cells in the feline (e.g., a feline having FIV or SELV).

In any of the methods described herein, the at least one agent or the at least one veterinary composition can be administered to the feline at least once a week (e.g., twice a week, three times a week, four times a week, once a day, twice a day, or three times a day). In some embodiments, at least one small molecule and/or at least one antibody and/or antigen binding fragment thereof is administered to the feline at least once a week (e.g., twice a week, three times a week, four times a week, once a day, twice a day, or three times a day). In some embodiments, at least two different agents are administered in the same composition (e.g., a solid composition or liquid composition). In some embodiments, at least two different agents are administered in two different compositions (e.g., a solid composition and a liquid composition). In some embodiments, the at least one agent is administered as a component of a feed composition (e.g., pellets, a liquid, or a semi-solid slurry).

In some embodiments, the feline can be administered the at least one agent or at least one veterinary composition over an extended period of time (e.g., over a period of at least 1 week, 2 weeks, 3 weeks, 1 month, 2 months, 3 months, 4 months, 5 months, 6 months, 7 months, 8 months, 9 months, 10 months, 11 months, or 12 months). A skilled veterinary profession may determine the length of treatment period using any of the methods described herein for diagnosing or following the effectiveness of retroviral treatment (e.g., the observation of at least one symptom of a retroviral infection in a feline or retroviral titers in the feline). As described herein, a skilled veterinary professional can also change the identity and number (e.g., increase or decrease) of agents administered to the feline and can also adjust (e.g., increase or decrease) the dosage or frequency of administration of at least one agent to the feline based on assessment of the effectivenss of retroviral treatment (e.g., using any of the methods described herein and known in the art).

In some embodiments, the feline can be administered the at least one agent or the at least one veterinary composition during a time of feline retroviral outbreak (e.g., starting at any time before or during the retroviral outbreak and ending at a time when the incidence of infected felines in the population has significantly decreased).

In some embodiments, the administration of at least one agent or at least one veterinary composition described herein does not cause detrimental immunosuppression in the feline. Determintal immunosuppression, for example, can be indicated by a nearly complete suppression of CD8⁺ T-cell activation (cytotoxic T-lymphocyte activation) in a feline following therapeutic treatment.

In some embodiments, the retroviral infection treated is FIV or FELV. In some embodiments, the virion or syncytium is FIV or FELV.

In some embodiments of the methods described herein, the feline may be further administered one or more (e.g., one, two, three, or four) additional therapeutic agents (e.g., a sedative, an analgesic, or an anti-inflammatory agent). The one or more additional therapeutic agents and the at least one agent (e.g., any of the agents described herein) can be administered in the same dose. In some embodiments, the one or more additional therapeutic agents and the at least one agent (e.g., any of the agents described herein) can be administered in separate dosage forms. In some embodiments, the one or more additional therapeutics can be administered to the feline prior to administering at least one agent (e.g., any of the agents described herein). In some embodiments, the one or more additional therapeutics can be administered to the feline after administering at least one agent (e.g., any of the agents described herein). In some embodiments, the one or more additional therapeutics and the at least one agent are administered to the feline such that there is overlap in the bioactive period of the one or more additional therapeutics and the at least one agent in the feline.

### Methods of Screening for Anti-Retroviral Agents

Also provided herein are methods of identifying candidate agents that can be useful for treating or decreasing the risk of retroviral infection in a feline, for decreasing virion entry (e.g., fusion) into a feline cell, for decreasing virion budding from a feline cell, or for decreasing syncytium transmission in a feline. These methods include providing LFA-1 and ICAM-1, and contacting the LFA-1 to ICAM-1 in the presence of the candidate agent, and determining the binding of LFA-1 to ICAM-1 in the presence of the candidate agent, where a decrease in the amount of binding between LFA-1 to ICAM-1 compared to the binding observed in the absence of the candidate agent indicates that the candidate agent can be useful for treating or decreasing the risk of retroviral infection in a feline, for decreasing virion entry into a feline cell, for decreasing virion budding from a feline cell, or for decreasing syncytium transmission in a feline.

In some embodiments of these methods, the LFA-1 may be expressed on the surface of a cell (e.g., a feline cell) and ICAM-1 is a recombinant soluble form of ICAM-1 protein. In some embodiments of these methods, the LFA-1 may be a recombinant form of soluble LFA-1 protein and ICAM-1 is expressed on the surface of a cell (e.g., a feline cell). In some embodiments, both LFA-1 and ICAM-1 are recombinant soluble proteins (e.g., and interactions identified by co-immunoprecipitation reactions).

In some embodiments of these methods, LFA -1 can be attached to a solid surface (e.g., a magnetic bead) and ICAM-1 is a recombinant soluble ICAM-1 protein. In some embodiments, ICAM-1 is attached to a solid surface (e.g., a magnetic bead) and LFA-1 is a recombinant soluble LFA-1 protein.

In some embodiments of any of the methods described herein, LFA-1 and/or ICAM-1 (including recombinant soluble forms of LFA-1 and/or ICAM-1) may be labelled (e.g., a fluorescent label, a radioisotope, or peptide-tag) for detection (e.g., detection by fluorescence, luminescence, or binding by a secondary antibody).

Candidate agents identified using any of the assays described herein can further tested in a feline model of retroviral infection. The efficacy of the candidate agent to treat or reduce the number, severity, duration, or frequency of one or more symptoms of a retroviral infection in a feline can be determined using any of the methods described herein.

### EXAMPLE

The invention is further described in the following examples, which do not limit the scope of the invention described in the claims. To the extent that the examples contain subject matter that falls outside the scope of the claims, the subject matter is included merely for reference purposes.

### Example 1. Antibodies that Specifically Bind to LFA-1 Decrease Retroviral Infection

An experiment was performed to determine whether an antibody that specifically binds to LFA-1 would decrease FIV infection in a FIV-susceptive, feline T-cell line (104-C1). In these experiments, FIV strain PPR ("PPR") was produced in 104-C1 cells. The resulting viral preparation was incubated with 0 µg/mL antibody (control), or 5 µg/mL or 10 µg/mL of anti-LFA1 antibody TS1-22 (produced from ATCC deposit number HB202), clone 25.3 (Becker Coulter (PN IM0157 and PN IM1433U), or clone 27 (Fisher Scientific No. 610826) for 30 minutes (at 37°C and 5% CO₂) before being added to 150,000 104-C1 cells, and spin inoculated for 2 hours at 3,000 rpm. After spinning, the cells were recovered for 3 hours at 37°C and 5% CO₂, before the viral media was replaced with fresh media containing the same concentrations of diluted antibody. After 6, 12, and 14 days of incubation in fresh medium, the cells were lysed and the reverse transcriptase activity in the cells was determined (counts per minute) as generally described in Thompson et al., PLoS One 2011; 6(8): e24020, 2011. The resulting data show that three different antibodies that specifically bind to LFA-1 significantly decrease FIV infection in feline T-cells (Figure 2). These data indicate that antibodies or antigen-binding fragments thereof that specifically bind to LFA-1, or its binding partner ICAM-1, can decrease retrovirus infection (e.g., FIV infection) in a feline and can reduce retrovirus virion entry (e.g., FIV virion entry) into a feline cell. These data further indicate that small molecules that decrease binding of LFA-1 to its binding partner ICAM-1 can decrease retrovirus infection (e.g., FIV infection) in a feline and can reduce retrovirus virion entry (e.g., FIV virion entry) into a feline cell.

### Example 2. Anti-CD11a Antibody and FIV Infection

Additional experiments may be performed to determine whether an anti-CD11a antibody would decrease or prevent FIV infection in feline cells. For example, in such experiments, TS1/22 antibody (anti-CD11a antibody) may be incubated with either feline peripheral blood mononuclear cells or the feline T-cell line 104-C1 and binding assayed by fluorescence-assisted cell sorting (FACS).

In additional experiments, feline 104-C1 T-cells may be left untreated or preincubated for 15 minutes with TS1-22 (10 µg/mL or 50 µg/mL). The cells may then be washed and infected with FIV-PPR. The presence of virus may be determined by measuring reverse transcriptase activity at days 4, 7, 10, and 14. In one set of controls, the cells may be left untreated with TS1/22 and uninfected with FIV.

## Claims

1. An antibody or antigen-binding fragment thereof that specifically binds to CD11a, for use in treating a retrovirus infection in a feline.

2. The antibody or antigen-binding fragment thereof that specifically binds to CD11a for use according to Claim 1, wherein the antibody or antigen-binding fragment thereof
(i) decreases LFA1 binding to ICAM-1, or
(ii) is an antibody that is a monoclonal antibody, or
(iii) contains at least one complementary determining region (CDR) from the light chain or the heavy chain of TS1/22 antibody (ATCC deposit number HB202);
and optionally -
(a) contains the three CDRs from the light chain of TS1/22 antibody or the three CDRs from the heavy chain of TS1/22 antibody, or
(b) contains the three CDRs from the light chain of TS1/22 antibody and the three CDRs from the heavy chain of TS1/22 antibody, optionally wherein the antibody or antigen-binding fragment thereof is TS1/22 antibody or an antigen-binding fragment of TS1/22 antibody.

3. The antibody or antigen-binding fragment thereof that specifically binds to CD11a for use according to Claim 1, wherein the antibody or antigen-binding fragment thereof
(i) is a feline antibody or an antigen-binding fragment thereof, or a felinized antibody or an antigen-binding fragment thereof, or
(ii) is a single-chain antibody, optionally wherein the single-chain antibody is a single-domain antibody, or
(iii) is selected from the group consisting of: a Fab fragment, a F(ab')2 fragment, and a scFv fragment.

4. The antibody or antigen-binding fragment thereof that specifically binds to CD11a for use according to Claim 1, wherein the antibody or antigen-binding fragment thereof is administered intravenously, intraarterially, ocularly, orally, subcutaneously, intraperitoneally, or intramuscularly.

5. The antibody or antigen-binding fragment thereof that specifically binds to CD11a for use according to any of Claims 1 to 4, wherein the feline is further administered at least one small molecule that decreases LFA-1 binding to ICAM-1
optionally wherein (i) the at least one small molecule and the antibody or antigen-binding fragment thereof are administered in the same composition, or (ii) the at least one small molecule and the antibody or antigen-binding fragment thereof are administered by different routes of administration.

6. The antibody or antigen-binding fragment thereof that specifically binds to CD11a for use according to Claim 1, wherein the administering results in a decrease in the severity, frequency, or duration of at least one symptom of a retroviral infection in the feline, or wherein the administering results in a decrease in retroviral titer in the feline, or
wherein the administering results in an increase in the ratio of CD4+ T-cells to CD8+ T-cells in the feline or wherein the administering does not result in detrimental immunosuppression in the feline.

7. The antibody or antigen-binding fragment thereof that specifically binds to CD11a for use according to Claim 1, wherein the antibody and/or antigen-binding fragment thereof is administered to the feline at least once a week, optionally at least once a day.

8. The antibody or antigen-binding fragment thereof that specifically binds to CD11a for use according to Claim 1, wherein the retroviral infection is feline immunodeficiency virus (FIV), or feline leukemia virus (FELV).

## Patentansprüche

1. Antikörper oder Antigenbindungsfragment davon, welcher/welches speziell an CD11a bindet, zur Verwendung bei der Behandlung einer Retrovirusinfektion bei einer Katze.

2. Antikörper oder Antigenbindungsfragment davon, welcher/welches speziell an CD11a bindet, zur Verwendung nach Anspruch 1, wobei der Antikörper oder das Antigenbindungsfragment davon
(i) LFA1-Bindung an ICAM-1 verringert, oder
(ii) ein Antikörper ist, welcher ein monoklonaler Antikörper ist, oder
(iii) wenigstens eine Komplementarität bestimmende Region (CDR) aus der Leichtkette oder der Schwerkette des TS1/22-Antikörpers (ATCC Hinterlegungsnummer HB202) enthält;
und wahlweise -
(a) die drei CDRs aus der Leichtkette des TS1/22-Antikörpers oder die drei CDRs aus der Schwerkette des TS1/22-Antikörpers enthält, oder
(b) die drei CDRs aus der Leichtkette des TS1/22-Antikörpers und die drei CDRs aus der Schwerkette des TS1/22-Antikörpers enthält, wahlweise wobei der Antikörper oder das Antigenbindungsfragment davon ein TS1/22-Antikörper oder ein Antigenbindungsfragment von einem TS1/22-Antikörper ist.

3. Antikörper oder Antigenbindungsfragment davon, welcher/welches speziell an CD11a bindet, zur Verwendung nach Anspruch 1, wobei der Antikörper oder das Antigenbindungsfragment davon
(i) ein feliner Antikörper oder ein Antigenbindungsfragment davon oder ein felinisierter Antikörper oder ein Antigenbindungsfragment davon ist, oder
(ii) ein Einzelketten-Antikörper ist, wahlweise wobei der Einzelketten-Antikörper ein Einzeldomänen-Antikörper ist, oder
(iii) aus der Gruppe ausgewählt ist, bestehend aus einem Fab-Fragment, einem F(ab')2-Fragment und einem scFv-Fragment.

4. Antikörper oder Antigenbindungsfragment davon, welcher/welches speziell an CD11a bindet, zur Verwendung nach Anspruch 1, wobei der Antikörper oder das Antigenbindungsfragment davon intravenös, intraarteriell, okulär, oral, subkutan, intraperitoneal oder intramuskulär verabreicht wird.

5. Antikörper oder Antigenbindungsfragment davon, welcher/welches speziell an CD11a bindet, zur Verwendung nach einem der Ansprüche 1-4, wobei der Katze des Weiteren wenigstens ein kleines Molekül, das LFA-1-Bindung an ICAM-1 verringert, verabreicht wird,
wahlweise wobei (i) das wenigstens eine kleine Molekül und der Antikörper oder das Antigenbindungsfragment davon in der gleichen Zusammensetzung verabreicht werden, oder (ii) das wenigstens eine kleine Molekül und der Antikörper oder das Antigenbindungsfragment davon über verschiedene Verabreichungsrouten verabreicht werden.

6. Antikörper oder Antigenbindungsfragment davon, das speziell an CD11a bindet, zur Verwendung nach Anspruch 1, wobei die Verabreichung zu einer Verringerung im Schweregrad, in der Häufigkeit oder Dauer von wenigstens einem Symptom einer Retrovirusinfektion bei der Katze führt, oder wobei die Verabreichung zu einer Reduktion des Retrovirentiters bei der Katze führt, oder
wobei die Verabreichung zu einem Anstieg in dem Verhältnis von CD4+-T-Zellen zu CD8+-T-Zellen in der Katze führt oder wobei die Verabreichung nicht zu einer nachteiligen Immunsuppression bei der Katze führt.

7. Antikörper oder Antigenbindungsfragment davon, das speziell an CD11a bindet, zur Verwendung nach Anspruch 1, wobei der Antikörper und/oder das Antigenbindungsfragment davon wenigstens einmal wöchentlich, wahlweise wenigstens einmal täglich an die Katze verabreicht wird.

8. Antikörper oder Antigenbindungsfragment davon, welcher/welches speziell an CD11a bindet, zur Verwendung nach Anspruch 1, wobei die Retrovirusinfektion eine Infektion mit dem felinen Immundefizienz-Virus (FIV) oder dem felinen Leukämie-Virus ist.

## Revendications

1. Anticorps ou fragment de liaison à l'antigène de celui-ci qui se lie spécifiquement à CD11a, à utiliser dans le traitement d'une infection rétrovirale chez un félin.

2. Anticorps ou fragment de liaison à l'antigène de celui-ci qui se lie spécifiquement à CD11a à utiliser selon la revendication 1, l'anticorps ou le fragment de liaison à l'antigène de celui-ci
(i) diminuant la liaison LFA1 à ICAM-1, ou
(ii) étant un anticorps qui est un anticorps monoclonal, ou
(iii) contenant au moins une région de détermination de la complémentaire (CDR) de la chaîne légère ou de la chaîne lourde de l'anticorps TS1/22 (N° de dépôt ATCC HB202) ;
et facultativement -
(a) contenant les trois CDR de la chaîne légère de l'anticorps TS1/22 ou les trois CDR de la chaîne lourde de l'anticorps TS1/22, ou
(b) contenant les trois CDR de la chaîne légère de l'anticorps TS1/22 et les trois CDR de la chaîne lourde de l'anticorps TS1/22, facultativement dans lequel l'anticorps ou le fragment de liaison à l'antigène de celui-ci est un anticorps TS1/22 ou un fragment de liaison à l'antigène de l'anticorps TS1/22.

3. Anticorps ou fragment de liaison à l'antigène de celui-ci qui se lie spécifiquement à CD11a à utiliser selon la revendication 1, l'anticorps ou le fragment de liaison à l'antigène de celui-ci
(i) étant un anticorps félin ou un fragment de liaison à l'antigène de celui-ci, ou un anticorps félinisé ou un fragment de liaison à l'antigène de celui-ci, ou
(ii) étant un anticorps à chaîne unique, facultativement dans lequel l'anticorps à chaîne unique est un anticorps à domaine unique, ou
(iii) étant choisi dans le groupe constitué : d'un fragment Fab, d'un fragment F(ab')2 et d'un fragment scFv.

4. Anticorps ou fragment de liaison à l'antigène de celui-ci qui se lie spécifiquement à CD11a à utiliser selon la revendication 1, l'anticorps ou le fragment de liaison à l'antigène de celui-ci étant administré par voie intraveineuse, intra-artérielle, oculaire, orale, sous-cutanée, intrapéritonéale ou intramusculaire.

5. Anticorps ou fragment de liaison à l'antigène de celui-ci qui se lie spécifiquement à CD11a à utiliser selon l'une quelconque des revendications 1 à 4, dans lequel on administre en outre au félin au moins une petite molécule qui diminue la liaison LFA-1 à ICAM-1
facultativement dans lequel (i) l'au moins une petite molécule et l'anticorps ou le fragment de liaison à l'antigène de celui-ci sont administrés dans la même composition, ou (ii) l'au moins une petite molécule et l'anticorps ou le fragment de liaison à l'antigène de celui-ci sont administrés par différentes voies d'administration.

6. Anticorps ou fragment de liaison à l'antigène de celui-ci qui se lie spécifiquement à CD11a à utiliser selon la revendication 1, dans lequel l'administration a pour résultat une diminution de la gravité, de la fréquence ou de la durée d'au moins un symptôme d'une infection rétrovirale chez le félin, ou dans lequel l'administration a pour résultat une diminution du titre rétroviral chez le félin, ou
dans lequel l'administration a pour résultat une augmentation du rapport entre les cellules T CD4+ et les cellules T CD8+ chez le félin ou dans lequel l'administration n'a pas pour résultat une immunosuppression nuisible chez le félin.

7. Anticorps ou fragment de liaison à l'antigène de celui-ci qui se lie spécifiquement à CD11a à utiliser selon la revendication 1, l'anticorps et/ou le fragment de liaison à l'antigène de celui-ci étant administré(s) au félin au moins une fois par semaine, facultativement au moins une fois par jour.

8. Anticorps ou fragment de liaison à l'antigène de celui-ci qui se lie spécifiquement à CD11a à utiliser selon la revendication 1, dans lequel l'infection rétrovirale est un virus de l'immunodéficience féline (FIV) ou un virus de la leucémie féline (FELV).
